(19) Europäisches Patentamt
European Patent Office
Office européen des brevets

(11) EP 3 954 698 A1

(12) **EUROPEAN PATENT APPLICATION**
published in accordance with Art. 153(4) EPC

(43) Date of publication:
**16.02.2022 Bulletin 2022/07**

(21) Application number: **20784736.9**

(22) Date of filing: **03.03.2020**

(51) International Patent Classification (IPC):
***C07K 7/06*** *(2006.01)* ***A61K 9/70*** *(2006.01)*
***A61L 15/22*** *(2006.01)* ***A61L 29/04*** *(2006.01)*
***A61K 47/30*** *(2006.01)* ***A61L 31/04*** *(2006.01)*
***A61L 27/14*** *(2006.01)*

(52) Cooperative Patent Classification (CPC):
**A61K 9/70; A61K 47/30; A61K 47/32; A61K 47/42; A61L 15/22; A61L 15/24; A61L 15/32; A61L 15/44; A61L 27/14; A61L 27/16; A61L 27/34; A61L 27/40; A61L 27/44; A61L 27/54; A61L 29/04;** (Cont.)

(86) International application number:
**PCT/JP2020/008958**

(87) International publication number:
**WO 2020/202987 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.04.2019 JP 2019071765**

(71) Applicants:
- **Hitachi, Ltd.**
  **Tokyo 100-8280 (JP)**
- **Tokyo Institute of Technology**
  **Tokyo 152-8550 (JP)**

(72) Inventors:
- **IWASAKI Tomio**
  **Tokyo 100-8280 (JP)**
- **MARUYAMA Masashi**
  **Tokyo 100-8280 (JP)**
- **SERIZAWA Takeshi**
  **Tokyo 152-8550 (JP)**
- **SAWADA Toshiki**
  **Tokyo 152-8550 (JP)**

(74) Representative: **MERH-IP Matias Erny Reichl Hoffmann**
**Patentanwälte PartG mbB**
**Paul-Heyse-Strasse 29**
**80336 München (DE)**

# (54) PEPTIDE EXHIBITING EXCELLENT ADHESION TO RESINS AND BIOCOMPATIBLE FUNCTIONAL MATERIAL USING SAME

(57) The present invention provides a biocompatible material improved in biocompatibility owing to adsorption of a peptide that has high adhesion with a resin and hardly separates from a surface of the resin, and a functional material including the biocompatible material. The biocompatible material (10) includes a resin (1) and a peptide (2) adsorbed on the resin (1). The resin (1) has methoxycarbonyl groups and methyl groups, and tryptophan or arginine accounts for 70% or more of amino acid residues in the peptide (2) . As an alternative, the resin 1 is a fluorinated resin, and 2,3,4,5,6-pentafluoro-phenylalanine, 3-(trifluoromethyl)alanine, serine, threonine, histidine, aspartic acid, glutamic acid, phenylalanine, or aspartic acid accounts for 40% or more of the amino acid residues in the peptide (2). The functional material includes the biocompatible material (10), and a functional substance. The functional substance is held on a surface of the biocompatible material (10) or is released from the surface of the biocompatible material (10).

FIG. 1

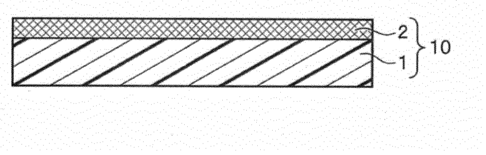


EP 3 954 698 A1

(52) Cooperative Patent Classification (CPC): (Cont.)
**A61L 29/08; A61L 29/12; A61L 29/16;**
**A61L 31/04; A61L 31/10; A61L 31/12;**
**A61L 31/16; C07K 7/06; C07K 7/08; C07K 17/00**

## Description

### Technical Field

The present invention relates to a peptide excellent in adhesion with a resin, and a biocompatible functional material, such as a biocompatible material or functional material, using the same.

### Background Art

In recent years, methods of medical treatment have diversified through developments in biotechnology in the medical field. When providing treatment or care for various diseases or injuries, use of therapeutic biodevices, artificial organs, and the like is spreading, leading to an increase in the likelihood of contact between living bodies and artificial materials. Further, a variety of medical devices, medical care products, hygiene products, and the like has been used in contact with skins.

An artificial material, upon contact with a living body, may induce various biological responses or reactions. If a device or artificial organ, or the like that uses an artificial material is implanted in the body or a device or medical care product, or the like that uses an artificial material remains in contact with a skin over a long term, various symptoms such as inflammations manifest through a foreign body reaction, an allergic reaction, or the like. There is accordingly an outstanding demand for materials having biocompatibility as materials for various devices, medical care products, and the like.

Conventionally, biocompatible materials with peptides bound to surfaces of organic materials, such as resins, or inorganic materials are known. If a peptide is bound to a surface of a resin as an artificial material or an inorganic material and covers the surface, the resin or inorganic material is improved in non-foreign body properties, non-irritant properties, interfacial compatibility, and the like, all of which are important for the conformity to living bodies. Search and designing are hence under way for peptides that specifically adsorb on artificial materials such as resins.

A peptide having an amino acid sequence represented by Glu-Leu-Trp-Arg-Pro-Thr-Arg (ELWRPTR) is disclosed, for example, Non-Patent Document 1. This peptide has high affinity with isotactic poly(methyl-methacrylate) resin (it-PMMA), shows a high value of 31 kJ/mol in terms of separation energy, and therefore indicates high adhesion with it-PMMA.

### Prior Art Document

### Non-Patent Document

Non-Patent Document 1: Takeshi Serizawa et al., Langmuir, 2007, 23(22), p. 11127 to 11133

### Summary of the Invention

### Problem to be Solved by the Invention

As described in Non-Patent Document 1, the adsorption of a peptide on a resin can efficiently impart biocompatibility by simple treatment or operation. If the affinity and adhesion between the resin and the peptide are not sufficiently high, however, the peptide may separate from the resin during use in a living body or a skin as an implanted site, resulting in contact between the resin and the living, so that an inflammation or the like may occur. If the peptide separates, a need arises for the replacement or the like of the material on which the peptide is no longer adsorbed. There is hence an outstanding desire for a peptide excellent in adhesion with a resin such that still higher separation energy than that of Non-Patent Document 1 is shown and biocompatibility has a longer durability life, and biocompatible function materials, such as biocompatible materials and functional materials, using the same.

The present invention therefore has as objects thereof the provision of a biocompatible material improved in biocompatibility owing to adsorption of a peptide that has high adhesion with a resin and hardly separates from a surface of the resin, and a functional material using the same.

### Means for Solving the Problem

The present inventors have conducted diligent research on peptides that show high affinity and adhesion with it-PMMA, and as a result, have found that tryptophan and arginine are prone to interaction with methoxy carbonyl groups and methyl groups of it-PMMA and are effective as amino acid residues that constitute peptides. It has also been found that proline and glutamic acid stabilize oxygen atoms of carbonyl groups, which exist in the backbone of a peptide, at

positions where the oxygen atoms tend to interact with methoxycarbonyl groups of it-PMMA and are hence effective as amino acid residues to be used in combination with tryptophan and arginine, thereby leading to completion of the present invention.

The present inventors also have conducted diligent research on peptides that show high affinity and adhesion with polytetrafluoroethylene (PTFE), and as a result, have found that 2,3,4,5,6-pentafluorophenylalanine, 3-trifluoromethylalanine, serine, threonine, histidine, aspartic acid, glutamic acid, phenylalanine, and asparagine are prone to interaction with molecular chains of PTFE and are effective as amino acid residues that constitute peptides, thereby leading to the completion of the present invention.

Specifically, for the solution of the above-described problem, a biocompatible material according to the present invention includes a resin, and a peptide adsorbed on the resin. The resin has methoxycarbonyl groups and methyl groups, and tryptophan or arginine for 70% or more of amino acid residues in the peptide.

Further, a functional material according to the present invention includes the above-described biocompatible material, and a functional substance that functions in a living body or on a surface of a living body. The functional substance is held on a surface of the biocompatible material, or is released from the surface of the biocompatible material.

## Advantage of the Invention

According to the present invention, there can be provided a biocompatible material improved in biocompatibility owing to adsorption of a peptide that has high adhesion with a resin and hardly separates from a surface of the resin, and a functional material including the biocompatible material.

## Brief Description of the Drawings

FIG. 1 is a view schematically illustrating a structure of a biocompatible material.
FIG. 2 is a diagram illustrating a correlation between calculated values of separation energy by a molecular dynamics simulation and measured values of separation energy as calculated based on a measurement experiment.
FIG. 3 is a diagram illustrating an example of a sensorgram obtained through measurement by a surface plasmon resonance method.
FIG. 4 is a sensorgram illustrating interaction between it-PMMA and an adsorptive peptide (RWWRPWW: SEQ ID NO. 1-1).
FIG. 5 is a sensorgram illustrating interaction between it-PMMA and an adsorptive peptide (RWWRRWW: SEQ ID NO. 1-2).
FIG. 6 is a sensorgram illustrating interaction between it-PMMA and an adsorptive peptide (EWWRPWR: SEQ ID NO. 1-4).
FIG. 7 is a sensorgram illustrating interaction between it-PMMA and an adsorptive peptide (RWWRPWR: SEQ ID NO. 1-5).
FIG. 8 is an image illustrating an atomic arrangement of the adsorptive peptide (RWWRPWW: SEQ ID NO. 1-1) adsorbed on it-PMMA.
FIG. 9 is an image illustrating an atomic arrangement of the adsorptive peptide (RWWRRWW: SEQ ID NO. 1-2) adsorbed on it-PMMA.
FIG. 10 is an image illustrating an atomic arrangement of the adsorptive peptide (EWWRPWR: SEQ ID NO. 1-4) adsorbed on it-PMMA.
FIG. 11 is an image illustrating an atomic arrangement of the adsorptive peptide (RWWRPWR: SEQ ID NO. 1-5) adsorbed on it-PMMA.
FIG. 12 is an image illustrating an atomic arrangement of an adsorptive peptide (ZZXXZZXXZZXX: SEQ ID NO. 2-1) adsorbed on PTFE.
FIG. 13 is an image illustrating an atomic arrangement of an adsorptive peptide (STSTSTSTSTST: SEQ ID NO. 2-7) adsorbed on PTFE.
FIG. 14 is an image illustrating an atomic arrangement of an adsorptive peptide (STSTSPSTSTST: SEQ ID NO. 2-3) adsorbed on PTFE.
FIG. 15 is an image illustrating an atomic arrangement of an adsorptive peptide (HHHHHHHHHHHH: SEQ ID NO. 2-11) adsorbed on PTFE.
FIG. 16 is an image illustrating an atomic arrangement of an adsorptive peptide (AAAAAAAAAAAA: SEQ ID NO. 4-4) adsorbed on PTFE.
FIG 17 is a diagram illustrating measurement results of contact angles of PTFE with adsorptive peptides adsorbed thereon.

FIG. 18 is a diagram illustrating results of dissociation of the adsorptive peptide from PTFE on which the adsorptive peptide has been adsorbed.

FIG. 19 is a diagram schematically illustrating a biocompatible material as a composite combined with a fibrous material.

FIG. 20 is a diagram schematically illustrating another biocompatible material as a composite combined with a filler material.

FIG. 21 is a diagram schematically illustrating a further biocompatible material laminated on a surface of an adherend.

FIG. 22 is a diagram schematically illustrating a still further biocompatible material that constitutes a housing for a device.

FIG. 23 is a diagram schematically illustrating a configuration example of a functional material.

FIG. 24 is a diagram schematically illustrating a configuration example of another functional material.

FIG. 25 is a diagram schematically illustrating a further configuration example of a further functional material.

Modes for Carrying Out the Invention

With reference to the figures, a description will hereinafter be made regarding peptides according to an embodiment of the present invention, biocompatible materials using the same, and functional materials using the same. In the individual figures to be referred to hereinafter, common elements are identified by the same reference signs, and their repeated descriptions are omitted.

<Biocompatible Materials>

FIG. 1 is a view schematically illustrating a structure of a biocompatible material.

As illustrated in FIG. 1, the biocompatible material 10 according to this embodiment includes at least a resin 1 as a main part of the material, and an adsorptive peptide (peptide) 2 adsorbed on the resin 1.

The biocompatible material 10 has a structure that the adsorptive peptide 2, a substance similar to biological substances, is adsorbed on the resin 1, and therefore is useful as a material that shows good biocompatibility compared with a case in which the resin 1 alone is used as a material. Throughout this specification, the term "biocompatibility" means a property of a material, by virtue of which it can coexist with a living body while fulfilling its inherent function without giving adverse effect or strong irritation to the living body over a long term.

The biocompatible material 10 can be used as a material for an optional article having a possibility of contact with a living body. Articles formed with the biocompatible material 10 are not particularly limited in kind, use, function, and so on insofar as they can be brought into contact with living bodies. The biocompatible material 10 is suitably used in a living body, for example, the human body, on a surface, for example, a skin of a living body, and for a use where the biocompatible material 10 comes into contact with another tissue or organ.

Specific examples of the articles formed with the biocompatible material 10 include artificial organs, artificial tissues, implantable therapeutic devices, epidermally fixed therapeutic devices, diagnostic devices, therapeutic appliances, surgical appliances, surgical materials, osteosynthesis materials, dental materials, filler materials, osteotomy appliances, orthodontic appliances, other prosthetic devices, contact lenses, eye glasses, clothing, footwear, wound dressings such as adhesive bandages and gauze, bandages, plasters, poultices, and so on.

The resin 1 that constitutes the biocompatible material 10 may be in any form such as a molded body or a non-molded solid body. The resin 1 can be formed in, but is not particularly limited to, an appropriate shape according to the kind, use, function, and the like of each article to be formed with the biocompatible material 10. The resin 1 may constitute the entire portion or only a portion of the article. As the resin 1, a resin having methoxycarbonyl groups and methyl groups or a fluorinated resin is used as will be described subsequently herein.

The adsorptive peptide 2, which constitutes the biocompatible material 10, is adsorbed to the resin 1 via non-covalent bonding, intermolecular forces such as van der Waals forces, hydrogen bonds, or dipole interactions, and is held on a surface of the resin 1. The adsorptive peptide 2 may be adsorbed on the entire area or a part of the surface of the resin 1. Used as the adsorptive peptide 2 is, as will be described subsequently herein, a peptide that has a predetermined amino acid sequence and shows specific affinity with the particular resin 1.

<Separation Energy>

The affinity (binding force) between a resin and an adsorptive peptide can be evaluated by the value of separation energy. Separation energy is defined as a difference in energy between a state in which the resin and the adsorptive peptide are adsorbed each other, and a state in which the resin and the adsorptive peptide are dissociated from each other.

An adsorption reaction (association reaction and dissociation reaction), in which a composite AB is formed through interaction between a resin A and a peptide B, can be expressed by the following reaction formula (1). In the

reaction formula (1):

[Chem. 1]

$$A+B \underset{k_{-1}}{\overset{k_1}{\rightleftarrows}} AB \quad \cdots (1)$$

where $k_1$ represents an association rate constant [$M^{-1} \cdot s^{-1}$], and $k_{-1}$ represents a dissociation rate constant [$s^{-1}$].

Assuming that the resin A and the peptide B follows a first order reaction, the rate equation of the adsorption reaction of the formation of the composite AB is expressed by the following equation (2):

[Math. 1]

$$\frac{d[AB]}{dt} = k_1[A][B] - k_{-1}[AB] \quad \cdots (2)$$

where [A] represents the concentration of the resin A, [B] represents the concentration of the peptide B, and [AB] represents the concentration of the composite AB, at a given time t.

Calculating with the left side of the equation (2) set at 0, the equilibrium constant Ka of the reaction formula (1) satisfies the following equation (3):

[Math. 2]

$$Ka = \frac{[AB]}{[A][B]} = \frac{k_{-1}}{k_1} \quad \cdots (3)$$

The standard Gibbs energy $\Delta G$ [J/mol] in the adsorption reaction of the formation of the composite AB is expressed by the following equation (4):
[Math. 3]

$$\Delta G = -RT\ln(Ka) \quad \cdots (4)$$

where R represents the gas constant R[$J \cdot K^{-1} \cdot mol^{-1}$], and T represents the absolute temperature [K].

The separation energy is equivalent to the standard Gibbs energy $\Delta G$ in the adsorption reaction of the formation of the composite AB, and therefore can be determined from the equations (3) and (4), using the association rate constant $k_1$ and the dissociation rate constant $k_{-1}$. Assuming the resin to be a ligand and a solution of the adsorptive peptide to be an analyte solution, the association rate constant $k_1$ and the dissociation rate constant $k_{-1}$ can be measured by a surface plasmon resonance method.

<PMMA-adsorbed Peptides>

First, a description will be made regarding an example of adsorbed peptides, specifically a PMMA-adsorbed peptide that shows high affinity with a resin having methoxycarbonyl groups and methyl groups such as poly(methyl methacrylate) resin (PMMA).

The PMMA-adsorbed peptide is formed of an amino acid sequence containing one or more amino acid residues

out of tryptophan (Trp: W), arginine (Arg: R), proline (Pro: P), and glutamic acid (Glu: E).

Table 1 shows the separation energies, association rate constants, and dissociation rate constants in the adsorption reactions between peptides and isotactic poly(methyl methacrylate) resin (it-PMMA), and the contents of tryptophan (W) and arginine (R) and the contents of proline (P) and glutamic acid (E) in amino acid sequences.

Table 1

| Peptide - it-PMMA | | | | | | | |
|---|---|---|---|---|---|---|---|
| Amino acid sequence | | Contents of amino acids W,R(P,E) | Separation energy SIM calculated value [kJ/mol] | Separation energy Measured value [kJ/mol] | Association rate constant [$M^{-1}\cdot s^{-1}$] | Dissociation rate constant [$10^{-3}\cdot s^{-1}$] | |
| 1-1 | RWWRPWW | 6/7 (1/7) | 41.1 | Unmeasurable | $1.5 \times 10^2$ | 0 (not dissociated) | Present Invention |
| 1-2 | RWWRRWW | 7/7 (0/7) | 38.1 | Unmeasurable | $1.6 \times 10^2$ | 0 (not dissociated) | Present Invention |
| 1-3 | WWWRPWW | 6/7 (1/7) | 37.2 | - | - | - | Present Invention |
| 1-4 | EWWRPWR | 5/7 (2/7) | 36.3 | Unmeasurable | $0.95 \times 10^2$ | 0 (not dissociated) | Present Invention |
| 1-5 | RWWRPWR | 6/7 (1/7) | 35.9 | Unmeasurable | $1.7 \times 10^2$ | 0 (not dissociated) | Present Invention |
| 1-6 | ELWRPTR | 3/7 (2/7) | 32.3 | 31.2 | 31 | 0.11 | Non-Patent Document 1 |
| 1-7 | EAWRPTR | 3/7 (2/7) | 31.8 | 30.9 | 30 | 0.12 | Non-Patent Document 1 |
| 1-8 | ELWRATR | 3/7 (1/7) | 23.7 | 22.6 | 3.4 | 0.38 | Non-Patent Document 1 |

| | | | | | | | |
|---|---|---|---|---|---|---|---|
| 1-9 | QLQKYPS | 0/7 (1/7) | 26.5 | 25.0 | 13 | 0.58 | |
| 1-10 | ELWRPAR | 3/7 (2/7) | 28.7 | 27.8 | 17 | 0.24 | |
| 1-11 | ELWR | 2/4 (1/4) | 19.1 | 18.4 | 2.5 | 3.00 | |
| 1-12 | ARPHLSF | 1/7 (1/7) | 28.5 | 27.6 | 13 | 0.20 | |
| 1-13 | SSPWMRE | 2/7 (2/7) | 28.3 | 27.5 | 15 | 0.24 | |
| 1-14 | GIRHTNR | 2/7 (0/7) | 27.9 | 26.5 | 20 | 0.48 | |
| 1-15 | ALWRPTR | 3/7 (1/7) | 30.7 | 29.3 | 31 | 0.11 | |
| 1-16 | ELARPTR | 2/7 (2/7) | 32.1 | 31.0 | 29 | 0.11 | |
| 1-17 | ELWAPTR | 2/7 (2/7) | 31.2 | 29.7 | 23 | 0.15 | |
| 1-18 | ELWRPTA | 2/7 (2/7) | 30.2 | 28.7 | 13 | 0.13 | |
| 1-19 | RPTR | 2/4 (1/4) | 28.1 | 26.7 | 7.4 | 0.16 | |

SEQ ID NOs. 1-1 to 1-5 are PMMA-adsorbed peptides according to the present invention, which have improved affinity with it-PMMA owing to molecular designing. SEQ ID NOs. 1-6 to 1-19 are the peptides described in Non-Patent Document 1 and having high affinity with it-PMMA.

In Table 1, there are shown, as separation energy, calculated values by a molecular dynamic simulation (SIM calculated values), and measured values calculated based on a measurement experiment. The measured values have been calculated from the equations (3) and (4), using the association rate constant $k_1$ and the dissociation rate constant $k_{-1}$ measured by the surface plasmon resonance method.

No measured values are available for SEQ ID NOs. 1-1 to 1-5, because the peptides have high affinity and no dissociation takes place. Measured values for SEQ ID NOs. 1-6 to 1-19 are calculated from the association rate constant $k_1$ and the dissociation rate constant $k_{-1}$ published in Non-Patent Document 1. The gas constant R is 8.314 [$m^2 \cdot kg \cdot s^{-2} \cdot K^{-1} \cdot mol^{-1}$], and the absolute temperature T is 300 [K] .

Using the reactive molecular dynamics calculation program "ReaxFF" as in Non-Patent Document (Akbar Bagri et al., Nature Chemistry, 2010, 2, p. 581 to 587), the molecular dynamics simulation was conducted by simulating behaviors of atoms and molecules in water. ReaxFF is a program that uses both a relation between bond distance and bond order and a relations between bond order and bond energy in a calculation, and is characterized in that it provides a reactive force field for describing bond breaking and formation in a hydrocarbon-oxygen system. In ReaxFF, non-bonded interactions are analyzed by conducting a calculation with the connectivity varied between all atom pairs.

The measurement experiment by the surface plasmon resonance method was conducted using an intermolecular interaction analysis system "Biacore X" (manufactured by GE Healthcare) as in Non-Patent Document (Morio Arai, "Analysis of Biomolecular Interactions by Biosensor (BIACORETM) Using Surface Plasmon Resonance," The Japanese Journal of Thrombosis and Hemostasis, The Japanese Society on Thrombosis and Hemostasis, October 1st, 1997, 8(5), p. 397 to 405).

Employed as it-PMMA was a resin having a number average molecular weight Mn of 32900, a weight average molecular weight Mw/number average molecular weight Mn ratio of 1.3., and a triad ratio mm:mr:rr of 97:3:0. it-PMMA was coated with a thickness of approximately 10 nm on a slide glass on which gold of a sensor chip was bonded, and the sensor chip coated with it-PMMA was installed in an analyzer.

As peptides, used were those which had the amino acid sequences shown in Table 1, had a free N-terminus, and were amidated at a C-terminus. Each peptide was purified by high-performance chromatography, and was then dissolved in 10 mM HEPES buffer (pH 7.4) with 150 mM of NaCl dissolved therein, so that an analyte solution was prepared.

In the measurement experiment by the surface plasmon resonance method, each analyte solution with the corresponding peptide dissolved therein was first allowed to flow at a temperature of 25°C and a flow rate of 20 μm/min for two minutes over a surface of it-PMMA, thereby inducing an association reaction. Subsequently, the buffer without containing the peptide was allowed to flow for two minutes under similar conditions, thereby inducing a dissociation reaction.

Sensorgrams, which had been measured at a plurality of concentrations different from one another, were then subjected to simultaneous global fitting, using analysis software "BIAevaluation ver. 4.1" (created by GE Healthcare), whereby the association rate constant $k_1$ and the dissociation rate constant $k_{-1}$ were determined from a linear relation between resonance units RU and time-dependent variations d (RU)/dt in resonance units, the linear relation corresponding to the equation (2).

As shown in Table 1, the association rate constants of SEQ ID NOs. 1-1 to 1-5 generally had large values compared with that of SEQ ID NO. 1-6 the separation energy of which was the largest in Non-Patent Document 1. On the other hand, the dissociation rate constants of SEQ ID NOs. 1-1 to 1-5 did not reach any finite value greater than 0 because the peptides had high affinity with it-PMMA and their dissociation was not observed.

From results of the molecular dynamics simulation and the measurement experiment, it is envisaged that SEQ ID NOs. 1-1 to 1-5 are prone to associate with it-PMMA but are hard to dissociate from it-PMMA. The measured values of the separation energy of SEQ ID NOs. 1-1 to 1-5 are evidently greater compared with that of SEQ ID NO. 1-6, thereby enabling to confirm the high degrees of their affinity with it-PMMA.

FIG. 2 is a diagram illustrating a correlation between calculated values of separation energy by the molecular dynamics simulation and measured values of separation energy as calculated based on the measurement experiment.

In FIG. 2, an axis of abscissa represents the calculated value [kJ/mol] of separation energy by the molecular dynamics simulation, whereas an axis of ordinate represents the measured value [kJ/mol] of separation energy as calculated based on the measurement experiment. In FIG. 2, the results of the peptides (SEQ ID NOs. 1-6 to 1-19) described in Non-Patent Document 1 are plotted.

As illustrated in FIG. 2, the calculated values of separation energy by the molecular dynamics simulation fit well to the measured values of separation energy as calculated based on the measurement experiment. From this result, it is envisaged that according to a program providing a reactive force field for describing bond breaking and formation like ReaxFF, the adsorption reaction between a resin and a peptide can be simulated with high accuracy.

Compared with SEQ ID NO. 1-6 the separation energy of which was the largest in Non-Patent Document 1, SEQ ID NOs. 1-1 to 1-5 are significantly improved in the calculated value of separation energy. SEQ ID NOs. 1-1 to 1-5 is therefore considered to be peptides that are high in affinity with it-PMMA and are excellent in adhesion with it-PMMA.

Next, measurement results of interactions between it-PMMA and a PMMA-adsorbed peptide by the surface plasmon resonance method will be described.

FIG. 3 is a diagram illustrating an example of a sensorgram obtained through measurement by the surface plasmon resonance method.

As illustrated in FIG. 3, signals through surface plasmon resonance show an increase when an analyte solution is allowed to flow over a sensor chip to have the PMMA-adsorbed peptide adsorbed on (associated with) it-PMMA. When a solution which does not contain the PMMA-adsorbed peptide is then allowed to flow, the PMMA-adsorbed peptide adsorbed on it-PMMA dissociates from it-PMMA, and signals through surface plasmon resonance hence show a decrease. From these results, the association rate constant $k_1$ and the dissociation rate constant $k_{-1}$ can be determined.

FIG. 4 is a sensorgram illustrating interaction between it-PMMA and the adsorptive peptide (RWWRPWW: SEQ ID NO. 1-1). FIG. 5 is a sensorgram illustrating interaction between it-PMMA and the adsorptive peptide (RWWRRWW: SEQ ID NO. 1-2). FIG. 6 is a sensorgram illustrating interaction between it-PMMA and the adsorptive peptide (EWWRPWR: SEQ ID NO. 1-4). FIG. 7 is a sensorgram illustrating interaction between it-PMMA and the adsorptive peptide (RWWRPWR: SEQ ID NO. 1-5).

As illustrated in each of FIGS. 4 to 7, with the PMMA-adsorbed peptide having the specific amino acid sequence, even when the solution with no PMMA-adsorbed peptide contained therein was allowed to flow, signals did not decrease but remained constant, and no dissociation of the PMMA-adsorbed peptide was observed. It is therefore considered that each PMMA-adsorbed peptide was hard to dissociate from it-PMMA, was required extremely high separation energy, and had sufficiently high affinity and adhesion between the resin and the peptide.

Next, results of atomic arrangements of it-PMMA and PMMA-adsorbed peptides as calculated by molecular dynamics simulations will be described.

FIG. 8 is an image illustrating an atomic arrangement of the adsorptive peptide (RWWRPWW: SEQ ID NO. 1-2) adsorbed on it-PMMA. FIG. 9 is an image illustrating an atomic arrangement of the adsorptive peptide (RWWRRWW: SEQ ID NO. 1-2) adsorbed on it-PMMA. FIG. 10 is an image illustrating an atomic arrangement of the adsorptive peptide (EWWRPWR: SEQ ID NO. 1-4) adsorbed on it-PMMA. FIG. 11 is an image illustrating an atomic arrangement of the adsorptive peptide (RWWRPWR: SEQ ID NO. 1-5) adsorbed on it-PMMA.

In each of FIGS. 8 to 11, an interface of it-PMMA, where the PMMA-adsorbed peptide is adsorbed, is shown as a three-dimensional modeling image as seen from a side of the PMMA-adsorbed peptide. Slim sticks indicate molecular chains of it-PMMA, bold sticks show a backbone of the adsorbed peptide, and medium sticks represent side chains of the adsorbed peptide. Largest spheres in the PMMA-adsorbed peptide indicate the oxygen atoms in carbonyl groups existing in the backbone.

On the image of each of FIGS. 8 to 11, hydrogen atoms in the PMMA-adsorbed peptide and water molecules of water of hydration and the like, which exist in the system, are set to non-display. In each sign indicated by an alphanumeric character, the left numeral is the number of the amino acid residue as counted from the N-terminus, and the right letter is the one letter abbreviation of the amino acid residue.

As illustrated in each of FIGS. 8 to 11, it-PMMA has a structure that molecular chains are oriented parallel to one another and methoxycarbonyl groups and methyl groups face each other. Between the molecular chains of it-PMMA, there are formed a region ($CH_3OCO$ region), in which methoxycarbonyl groups are located primarily, and another region ($CH_3$ region), in which methyl groups are located primarily.

On the other hand, the backbone of the PMMA-adsorbed peptide is oriented substantially parallel to the molecular chains of it-PMMA, and forms a zigzag structure in a β-strand-like shape. The oxygen atoms (largest spheres) of the carbonyl groups existing in the backbone of the PMMA-adsorbed peptide are each located on a side of a proximal end of the facing methoxycarbonyl group (in a neighborhood of a facing ester group) in it-PMMA.

Further, the side chains of the amino acid residues located at odd-numbered positions as counted from the N-terminus toward the C-terminus, among the amino acid residues constituting the PMMA-adsorbed peptide, are located in the $CH_3OCO$ region, and are close to methoxycarbonyl groups of another it-PMMA adjacent it-PMMA that is closest to the backbone of the PMMA-adsorbed peptide.

On the other hand, the side chains of the amino acid residues located at even-numbered positions as counted from the N-terminus toward the C-terminus, among the amino acid residues constituting the PMMA-adsorbed peptide, are located in the $CH_3$ region, and are close to the backbone and methyl groups of the it-PMMA located adjacent and on an opposite side of it-PMMA that is closest to the backbone of the PMMA-adsorbed peptide.

According to the simulation results illustrated in each of FIGS. 8 to 11, the backbone of the PMMA-adsorbed peptide is considered to form intermolecular interactions with the molecular chains of it-PMMA. Further, the side chains of the amino acid residues located at the odd-numbered positions as counted from the N-terminus toward the C-terminus, among the amino acid residues constituting the PMMA-adsorbed peptide, are considered to form intermolecular interactions, such as hydrogen bonds with neighborhoods of methoxycarbonyl groups of the it-PMMA, the methoxycarbonyl groups being adjacent it-PMMA that is closest to the backbone of the PMMA-adsorbed peptide. On the other hand, the side chains of the amino acid residues located at the even-numbered positions as counted from the N-terminus toward the C-terminus, among the amino acid residues constituting the PMMA-adsorbed peptide, form intermolecular interactions with a backbone and methyl groups of the it-PMMA, the backbone and methyl groups being adjacent the it-PMMA that is closest to the backbone of the PMMA-adsorbed peptide.

Especially according to the simulation results illustrated in FIGS. 8, 10 and 11, it is envisaged that, if proline and/or glutamic acid exists in a PMMA-adsorbed peptide, the zigzag structure of the backbone of the PMMA-adsorbed peptide becomes resistant to disruption and the structure of the backbone and the orientation of side chains are stabilized.

Based on the results of Table 1 and FIGS. 8 to 11, tryptophan and arginine are considered to be effective as amino acid residues constituting a PMMA-adsorbed peptide in that they have high adsorption forces with methoxycarbonyl groups and methyl groups. Further, proline and glutamic acid are also considered to be effective in that they contribute to the stabilization of the structure of the backbone and the orientation of the side chains of the PMMA-adsorbed peptide, although their adsorption forces are inferior to those of tryptophan and arginine.

Therefore, the PMMA-adsorbed peptide preferably contains one or more amino acid residues among tryptophan (W), arginine (R), proline (P), and glutamic acid (E), with the content of tryptophan or arginine being high.

The PMMA-adsorbed peptide may also be formed using only W, R, P, and E, or may also be formed using other amino acid residues. The amino acid residues that constitute the PMMA-adsorbed peptide are each of the L-type, preferably.

The PMMA-adsorbed peptide has separation energy of preferably 35.0 kJ/mol or higher, more preferably 36.0 kJ/mol or higher, still more preferably 37.0 kJ/mol or higher, even more preferably 38.0 kJ/mol or higher.

The PMMA-adsorbed peptide has a length of preferably 5 or more amino acid residues, more preferably 6 or more amino acid residues. On the other hand, 100 or fewer amino acid residues are preferred, 20 or fewer amino acid residues are more preferred, 15 or fewer amino acid residues are still more preferred, and 10 or fewer amino acid resides are even more preferred. The longer the PMMA-adsorbed peptide, the higher affinity can be obtained with the resin. On the other hand, the shorter the PMMA-adsorbed peptide, the harder intramolecular or intermolecular aggregations take place, the easier the alterations such as chemical modifications and clustering. Particularly preferably, the PMMA-adsorbed peptide has a length of 7 amino acid residues.

In the PMMA-adsorbed peptide, it is preferred that tryptophan or arginine accounts for 70% or more of the amino acid residues. The content of each of tryptophane and arginine in the total length of the amino acid sequence is more preferably 75% or higher, still more preferably 80% or higher, even more preferably 85% or higher, yet more preferably 90% or higher. On the other hand, the content of tryptophane or arginine may be set at 100%, lower than 95%, or lower than 90%.

Tryptophane and arginine have relatively long side chains, and can form relatively strong interactions with methoxycarbonyl groups and methyl groups of the resin. Further, the side chain of tryptophan is relatively rigid, and tends to stabilize the structure of the backbone and the orientation of the side chains of the PMMA-adsorbed peptide. On the other hands, arginine has longer side chains than tryptophan, and therefore can form interactions different from those formed by tryptophan. If the content of tryptophan or arginine is increased, a PMMA-adsorbed peptide that shows high affinity with a resin having methoxycarbonyl groups and methyl groups is obtained.

In the PMMA-adsorbed peptide, it is preferred that proline or glutamic acid preferably accounts for 10% or more of the amino acid residues. The content of proline or glutamic acid in the total length of the amino acid sequence may be set at 15% or higher, or 20% or higher. Further, the content of proline or glutamic acid may be set at lower than 25%, lower than 20%, or lower than 15%.

Proline has a ring structure, so that the bond angle and dihedral angle of a peptide bond are restricted or fixed. Proline therefore exhibits an action to stabilize the structure of the backbone. On the other hand, glutamic acid has a relatively long side chain, and contains a polar group in the side chain. If proline or glutamic acid is interposed, the zigzag structure in a β-strand-like shape becomes resistant to disruption, and the structure of the backbone and the orientation of side chains may be stabilized.

Among the amino acid residues constituting the PMMA-adsorbed peptide, the amino acid residues located at the odd-numbered positions as counted from the N-terminus toward the C-terminus in the PMMA-adsorbed peptide and the amino acid residues located at the even-numbered positions, both, as counted from the N-terminus toward the C-terminus in the PMMA-adsorbed peptide preferably do not have a sequence containing two or more consecutive amino acid residues of the same type.

In the case of a sequence containing two or more consecutive amino acid residues of the same type, for example, like Trp-Xaa-Trp-Xaa-Trp (Xaa is an optional amino acid residue), odd-numbered side chains themselves and even-numbered side chains themselves interfere, so that the structure of the backbone is disrupted, or aggregations occur through intramolecular interactions. If formed in a sequence containing no consecutive amino acid residues of the same type, on the other hand, the structure of the backbone and the orientation of side chains in the PMMA-adsorbed

peptide tend to be stabilized.

In the PMMA-adsorbed peptide, the even-numbered amino acid residues as counted from the N-terminus toward the C-terminus of the PMMA-adsorbed peptide may preferably have a sequence with Trp, Arg, and Trp in this order (Trp-Xaa-Arg-Xaa-Trp) or a sequence with Arg, Trp, and Arg in this order (Arg-Xaa-Trp-Xaa-Arg) (where the third amino acid residue is the last even-numbered amino acid residue located on the side of the C-terminus, or the next even-numbered amino acid residue is an intermediate amino acid residue that is an amino acid residue of a different type).

With such a sequence, amino acid residues of the same type do not follow continuously, the structure of the backbone and the orientation of the side chains of the PMMA-adsorbed peptide are stabilized, and interactions tend to occur between the side chains of the amino acid residues and the methyl groups of the resin. Such a sequence therefore tends to provide high affinity with the resin.

It is particularly preferred for the PMMA-adsorbed peptide that the 2n-th amino acid residue, the (2n+2)th amino acid residue, and the (2n+4)th amino acid residue, all, as counted from the N-terminus are tryptophan (W), arginine (R), and tryptophan (W), respectively (where n represents a natural number, and the (2n+4)th amino acid residue is the last even-numbered amino acid residue located on the side of the C-terminus or the next (2n+6)th amino acid residue is an intermediate amino acid residue that is an amino acid residue other than arginine).

In general, the even-numbered amino acid residues of the PMMA-adsorbed peptide come close to methyl groups of it-PMMA, thereby stabilizing the PMMA-adsorbed peptide. However, the side chain of arginine is long compared with a methyl group, so that the side chains of even-numbered arginine residues are prone to bending and tend to hardly orient toward adjacent molecular chains of the resin. If the even-numbered amino acid residues have a sequence of Arg, Trp, and Arg in this order, the side chains of arginine may interfere with one another across the adjacent even-numbered amino acid residue, so that the structure of the backbone of the PMMA-adsorbed peptide tends to be disrupted. If the even-numbered amino acid residues have a sequence of Trp, Arg, and Trp in this order, on the other hand, the structure of the backbone and the orientation of the side chains of the PMMA-adsorbed peptide can be stabilized while increasing the affinity with the resin.

It is preferred for the PMMA-adsorbed peptide that the (2m-1)th amino acid residue, the (2m+1)th amino acid residue, and the (2m+3)th amino acid residue, all, as counted from the N-terminus are arginine (R), tryptophan (W), and proline (P), respectively, the (2m-1)th amino acid residue, the (2m+1)th amino acid residue, and the (2m+3)th amino acid residue, all, as counted from the N-terminus are arginine (R), tryptophan (W), and arginine (R), respectively, the (2m-1)th amino acid residue, the (2m+1)th amino acid residue, and the (2m+3)th amino acid residue, all, as counted from the N-terminus are tryptophan (W), tryptophan (W), and proline (P), respectively, or the (2m-1)th amino acid residue, the (2m+1)th amino acid residue, and the (2m+3)th amino acid residue, all, as counted from the N-terminus are glutamic acid (E), tryptophan (W), and proline (P), respectively (where m represents a natural number).

In general, the odd-numbered amino acid residues of the PMMA-adsorbed peptide come close to methoxycarbonyl groups of it-PMMA, thereby stabilizing the PMMA-adsorbed peptide. However, a methoxycarbonyl group is long compared with a methyl group. Even if the odd-numbered amino acid residues have a sequence of Arg, Trp, and Arg in this order, side chains of the arginine residues rarely interfere with each other across the adjacent odd-numbered amino acid residue, so that the structure of the backbone of the PMMA-adsorbed peptide tends to be hardly disrupted. If the odd-numbered amino acid residues have the sequence of Arg, Trp, and Arg in this order, the side chains of arginine, which are longer than those of tryptophan, form intermolecular interactions, so that the affinity with the resin can be increased.

It is more preferred for the PMMA-adsorbed peptide that, when the (2m+3)th amino acid residue as counted from the N-terminus is proline (P), the (2m+5)th amino acid residue is tryptophan (R) or arginine (R).

If the (2m+5)th amino acid residue is Trp or Arg when the (2m+3)th amino acid residue is Pro, the zigzag structure of the backbone of the PMMA-adsorbed peptide is stabilized by the molecular structure of Pro, thereby tending to have the side chain of (2m+5)th Trp or Arg oriented toward the side of the methoxycarbonyl group of the adjacent molecular chain. The affinity with the resin may hence be increased if the (2m+5)th amino acid residue is Trp or Arg.

The PMMA-adsorbed peptide may have such a predetermined even-numbered amino acid sequence (W·R·W, R·W·R) or such a predetermined odd-numbered amino acid sequence (R·W·P, R·W·R, W·W·P, E·W·P) on a side closest to the N-terminus among all the amino acid residues, or at an intermediate position among all the amide acid residues. In other words, as the above-described numbers of the amino acid residues, n = 1 and m = 1 may be acceptable, or n > 1 and m > 1 may be acceptable. However, the PMMA-adsorbed peptide is preferably as short as 10 or fewer amino acid residues. In this case, the PMMA-adsorbed peptide may preferably have such a predetermined amino acid sequence on the side closest to the N-terminus among all the amino acid residues (amino acid residues of n = 1 or m = 1).

The PMMA-adsorbed peptide may also have one or a plurality of such predetermined even-numbered amino acid sequences (W·R·W, R·W·R) or such predetermined odd-numbered amino acid sequences (R·W·P, R·W·R, W·W·P, E·W·P) in the total length. However, the PMMA-adsorbed peptide is preferably as short as 10 or fewer amino acid residues. In this case, the PMMA-adsorbed peptide may preferably have a single unit of such a predetermined amino acid sequence in the total length.

The PMMA-adsorbed peptide may also have such a predetermined even-numbered amino acid sequences (W·R·W, R·W·R) and such a predetermined odd-numbered amino acid sequence (R·W·P, R·W·R, W·W·P, E·W·P) at positions consecutive to each other or at positions non-consecutive to each other. In other words, as the numbers of the above-described amino acid residues, n = m may be acceptable, or n ≠ m may be acceptable. However, the PMMA-adsorbed peptide is preferably as short as 10 or fewer amino acid residues. In this case, the PMMA-adsorbed peptide may preferably have such predetermined amino acid sequences at positions consecutive to each other (amino acid residues of n = m).

The PMMA-adsorbed peptide is preferably a peptide containing an amino acid sequence represented by any one of the following SEQ ID NOs. (1-1) to (1-5) with one or two amino acid residues added thereto, inserted thereinto, substituted therefor, or deleted therefrom, more preferably a peptide containing an amino acid sequence represented by any one of the following SEQ ID NOs. (1-1) to (1-5), particularly preferably an amino acid sequence represented by any one of the following SEQ ID NOs. (1-1) to (1-5).

RWWRPWW... (1-1)
RWWRRWW... (1-2)
WWWRPWW...(1-3)
EWWRPWR... (1-4)
RWWRPWR...(1-5)

The PMMA-adsorbed peptide may also have an amino acid sequence having a specific function, in addition to the amino acid sequence represented by any one of SEQ ID NOs. 1-1 to 1-5. The PMMA-adsorbed peptide may have such an amino acid sequence on a side closest to the N-terminus among all the amino acid residues, or may have it at an intermediate position among all the amino acid sequences.

The PMMA-adsorbed peptide may also have one or a plurality of amino acid sequences of SEQ ID NOs. 1-1 to 1-5 in the total length. The PMMA-adsorbed peptide may have a single kind or plural types of these amino acid sequences.

The N-terminus and C-terminus of the PMMA-adsorbed peptide may be chemically modified, or may be in an optional ionization state. For example, the N-terminus may be any one of $-NH_2$, $-NH_3+$, $-CH_3CO$, 9-fluorenylmethyloxy-carbonyl (Fmoc) group, tert-butoxycarbonyl (Boc) group, or the like. The C-terminus may be any one of -COOH, $-COO^-$, $-CONH_2$, $-CONH_3^+$, or the like.

If the PMMA-adsorbed peptide is a peptide containing an amino acid sequence represented by any one of SEQ ID NOs. 1-1 to 1-5 or an amino acid sequence similar to the above-mentioned amino acid sequence, the separation energy relative to a resin having methoxycarbonyl groups and methyl groups has a higher theoretical value, so that high affinity is obtained with the resin. Accordingly, the peptide adsorbed on the resin hardly undergoes separation, thereby enabling to obtain a biocompatible material the biocompatibility of which lasts over a longer term.

The PMMA-adsorbed peptide can be synthesized using a variety of synthesis methods including, for example, chemical synthesis methods such as liquid-phase synthesis methods and solid-phase synthesis methods, genetic engineering synthesis methods, and the like. Usable as liquid-phase synthesis methods are, for example, the synthesis method using a soluble anchor, which is described in Non-Patent Document (Keisuke Aihara et al., Organic Letters, 2015, 17(3), p. 696 to 699), and the stepwise method, the fragment condensation method, and the like, which are described in Non-Patent Document (Noboru Yanaihara, and two others, "Chemical Syntheses of Peptides and Their Applications," Journal of Synthetic Organic Chemistry, The Society of Synthetic Organic Chemistry, Japan, November 1st, 1998, 46(11), p. 1073 to 1084). On the other hand, usable as the solid-phase synthesis methods are, for example, the various types of synthesis methods described in the above-described Non-Patent Document (Journal of Synthetic Organic Chemistry), and various types of synthesis methods described in Non-Patent Document (Kiyoshi Nokihara, "New Technology for Peptide Syntheses," KOBUNSHI (in Japanese), The Society of Polymer Science, Japan, September 1st, 1994, 43(9), p. 611 to 615).

<Resin Having Methoxycarbonyl Groups and Methyl Groups>

As the resin on which the PMMA-adsorbed peptide is to be adsorbed (the resin 1 that constitutes the biocompatible material 10), any optional resin can be used insofar as it is a resin having methoxycarbonyl groups and methyl groups.

The resin on which the PMMA-adsorbed peptide is to be adsorbed may be a polymer of a monomer having a methoxycarbonyl group and a methyl group, a copolymer of a monomer having a methoxycarbonyl group and a monomer having a methyl group, or a copolymer of a monomer having a methoxycarbonyl group and a methyl group and another monomer. The copolymer may be any one of a block copolymer, a random copolymer, and a graft copolymer.

As the resin on which the PMMA-adsorbed peptide is to be adsorbed, poly(methyl methacrylate) resin (PMMA) is preferred, with isotactic poly(methyl methacrylate) resin (it-PMMA) being particularly preferred. If the resin is PMMA, the monomer has a methoxycarbonyl group and a methyl group, so that high affinity is available. If the resin is it-PMMA, on the other hand, methoxycarbonyl groups and methyl groups are arranged in a stereoregular and uniform conformation, so that higher affinity is available. Further, it-PMMA also facilitates processing of the biocompatible material as it has a lower glass transition temperature compared with atactic poly(methyl-methacrylate) resin (at-PMMA) or syndiotactic poly(methyl-methacrylate) resin(st-PMMA).
Isotactic poly(methyl methacrylate) resin (it-PMMA) has an isotactic triad fraction (mm) of 50% or higher, preferably 70% or higher, more preferably 90% or higher, still more preferably 95% or higher. it-PMMA may also contain a monomer other than methyl methacrylate.
Poly(methyl methacrylate) resin (PMMA) can be synthesized using, for example, the synthesis method described in Non-Patent Document 1. it-PMMA is obtained if methyl methacrylate is subjected to anionic polymerization in a non-polar solvent using as an initiator a Grignard reagent having a bulky substituent group such as a tert-butyl group. As the non-polar solvent, diethyl ether, dichloromethane, toluene, or the like can be used, for example.

<PTFE-adsorbed Peptide>

As an example of the adsorptive peptide, a description will next be made regarding a PTFE-adsorbed peptide that shows high affinity with polytetrafluoroethylene resin (PTFE) or the like.
The PTFE-adsorbed peptide is formed of an amino acid sequence containing one or more types of amino acid residues among 2,3,4,5,6-pentafluorophenylalanine (Phe(5F): represented by Z), 3-(trifluoromethyl)alanine (2-amino-4,4,4-trufluorobutyric acid)(Abu(3F): represented by X), serine (Ser: S), threonine (Thr: T), histidine (His: H), aspartic acid (Asp: D), glutamic acid (Glu: E), phenylalanine (Phe: F), asparagine (Asn: N), and proline (Pro: P).
Table 2 shows separation energies of adsorption reactions between peptides and polytetrafluoroethylene resin (PTFE), and the contents of 2,3,4,5,6-pentafluorophenyl-alanine (Z), 3-(trifluoromethyl)alanine (X), serine (S), threonine (T), histidine (H), aspartic acid (D), glutamic acid (E), phenylalanine (F), and asparagine (N) in their amino acid sequences.

Table 2

| Peptide - PTFE | | | |
|---|---|---|---|
| Amino acid sequence | | Content of amino acid residues Z, X, S, T, H, D, E, F, N | Separation energy SIM calculated value [kJ/mol] |
| 2-1 | ZZXXZZXXZZXX | 12/12 | 65.2 |
| 2-2 | ZXXZZXXZZXXZ | 12/12 | 65.1 |
| 2-3 | STSTSPSTSTST | 11/12 | 40.4 |
| 2-4 | TSTSTPTSTSTS | 11/12 | 40.3 |
| 2-5 | SSSSSPSSSSSS | 11/12 | 40.3 |
| 2-6 | TTTTTPTTTTTT | 11/12 | 40.2 |
| 2-7 | STSTSTSTSTST | 12/12 | 39.1 |
| 2-8 | TSTSTSTSTSTS | 12/12 | 39.0 |
| 2-9 | SSSSSSSSSSSS | 12/12 | 39.0 |
| 2-10 | TTTTTTTTTTTT | 12/12 | 38.9 |
| 2-11 | HHHHHHHHHHHH | 12/12 | 38.9 |
| 2-12 | HHHHHPHHHHHH | 11/12 | 38.7 |
| 2-13 | DSDSDPDSDSDS | 11/12 | 38.5 |
| 2-14 | DSDSDSDSDSDS | 12/12 | 37.3 |
| 2-15 | DHDHDHDHDHDH | 12/12 | 37.1 |
| 2-16 | DHDHDPDHDHDH | 11/12 | 36.9 |
| 2-17 | DEDEDPDEDEDE | 11/12 | 36.2 |
| 2-18 | DEDEDEDEDEDE | 12/12 | 35.6 |

(continued)

| Peptide - PTFE | | | |
|---|---|---|---|
| Amino acid sequence | | Content of amino acid residues Z, X, S, T, H, D, E, F, N | Separation energy SIM calculated value [kJ/mol] |
| 2-19 | FHHFFHHFFHHF | 12/12 | 34.6 |
| 2-20 | FFHHFFHHFFHH | 12/12 | 34.4 |
| 2-21 | NENENPNENENE | 11/12 | 34.2 |
| 2-22 | NENENENENENE | 12/12 | 34.0 |
| 3-1 | VHFPTKISEGDM | 6/12 | 28.8 |
| 3-2 | TFTLNSVHRSVH | 8/12 | 27.8 |
| 3-3 | SPHLHTSSPWER | 7/12 | 27.4 |
| 3-4 | FIESKTPVDPDG | 6/12 | 27.1 |
| 3-5 | GSESRTLFHPEG | 7/12 | 27.0 |
| 3-6 | EALTVNIKREME | 5/12 | 26.9 |
| 3-7 | SMIVEPRMLSTH | 5/12 | 25.9 |
| 4-1 | SAAASAAASAAS | 4/12 | 19.4 |
| 4-2 | HAAAHAAAHAAH | 4/12 | 19.2 |
| 4-3 | YYYYYYYYYYYY | 0/12 | 18.9 |
| 4-4 | AAAAAAAAAAAA | 0/12 | 18.3 |

In Table 2, SEQ ID NOs. 2-1 to 2-22 are PTFE-adsorbed peptides according to the present invention, which have improved affinity with PTFE owing to molecular designing. SEQ ID NOs. 3-1 to 3-7 are PTFE-adsorbed peptides according to the present invention, which have been acquired through random screening based on their affinity with PTFE. SEQ ID NOs. 4-1 to 4-4 are 12-mer peptides prepared as comparison.

In Table 2, as separation energy, calculated values (SIM calculated values) by molecular dynamics simulations are shown. Using the reactive molecular dynamics calculation program "ReaxFF" as for the PMMA-adsorbed peptides, the molecular dynamics simulations have been each conducted by simulating behaviors of atoms and molecules in water.

As shown in Table 2, the contents of one or more of the nine types of amino acids in SEQ ID NOs. 2-1 to 2-22 and SEQ ID NOs. 3-1 to 3-7 are 40% or higher, thereby indicating high separation energy. Comparing SEQ ID NOs. 3-1 to 3-7 in which the contents of one or more of the nine types of amino acids are 40% or higher and 70% or lower with SEQ ID NOs. 2-1 to 2-22 in which the content of one or more of the nine types of amino acids is 90% or higher, the latter indicate higher separation energy. In SEQ ID NOs. 4-1 to 4-4, on the other hand, the contents of one or more of the nine types of amino acids are lower than 40%, thereby indicating low separation energy.

From the results of the molecular dynamics simulations, it is envisaged that a PTFE-adsorbed peptide, which contains one or more of the nine types of amino acids at a high content or higher contents and has an appropriate recurring structure, shows high affinity with PTFE.

Next, calculation results of atomic arrangements of PTFE and PTFE-adsorbed peptides by molecular dynamics simulations will be described.

FIG. 12 is an image illustrating an atomic arrangement of the adsorptive peptide (ZZXXZZXXZZXX: SEQ ID NO. 2-1) adsorbed on PTFE. FIG. 13 is an image illustrating an atomic arrangement of the adsorptive peptide (STSTSTSTSTST: SEQ ID NO. 2-7) adsorbed on PTFE. FIG. 14 is an image illustrating an atomic arrangement of the adsorptive peptide (STSTSPSTSTST: SEQ ID NO. 2-3) adsorbed on PTFE. FIG. 15 is an image illustrating an atomic arrangement of the adsorptive peptide (HHHHHHHHHHHH: SEQ ID NO. 2-11) adsorbed on PTFE. FIG. 16 is an image illustrating an atomic arrangement of the adsorptive peptide (AAAAAAAAAAAA: SEQ ID NO. 4-4) adsorbed on PTFE.

In each of FIGS. 12 to 16, an interface of PTFE, where the PTFE-adsorbed peptide is adsorbed, is shown as a three-dimensional modeling image as seen from a side of the PTFE-adsorbed peptide. Slim sticks indicate molecular chains of PTFE, bold sticks show a backbone of the adsorbed peptide, and medium sticks represent side chains of the adsorbed peptide. Largest spheres in the PTFE-adsorbed peptide indicate the oxygen atoms in carbonyl groups existing in the backbone.

On the image of each of FIGS. 12 to 16, hydrogen atoms in the PTFE-adsorbed peptide and water molecules of water of hydration and the like, which exist in the system, are set to non-display. In each sign indicated by alphanumerics, the left numeral is the number of the amino acid residue as counted from the N-terminus, and the right letter is one letter abbreviation of the amino acid residue.

As illustrated in each of FIGS. 12 to 16, molecular chains are oriented parallel to one another in PTFE. On the other hand, the backbone of the PTFE-adsorbed peptide is oriented substantially parallel to the molecular chains of PTFE, and forms a zigzag structure in a β-strand-like shape. The oxygen atoms (largest spheres) of the carbonyl groups existing in the backbone of the PTFE-adsorbed peptide are each located in a neighborhood of the molecular chain of PTFE.

Further, the side chains of the amino acid residues located at odd-numbered positions as counted from the N-terminus toward the C-terminus, among the amino acid residues constituting the PTFE-adsorbed peptide, are close to another molecular chain of the PTFE, the another molecular chain being adjacent the PTFE that is closest to the backbone of the PTFE-adsorbed peptide.

On the other hand, the side chains of the amino acid residues located at even-numbered positions as counted from the N-terminus toward the C-terminus, among the amino acid residues constituting the PTFE-adsorbed peptide, are close to a further molecular chain of the PTFE, the further molecular chain being adjacent and on an opposite side of the PTFE that is closest to the backbone of the PTFE-adsorbed peptide.

According to the simulation results illustrated in each of FIGS. 12 to 15, the backbone of the PTFE-adsorbed peptide is considered to form intermolecular interactions with molecular chains of the PTFE. Further, the side chains of the amino acid residues which constitute the PTFE-adsorbed peptide are considered to form intermolecular interactions with a plurality of other molecular chains of the PTFE, the plurality of other molecular chains being adjacent the PTFE that is closest to the backbone of the PTFE-adsorbed peptide.

Especially according to the simulation results illustrated in FIG. 14, it is envisaged that, if proline exists in a PTFE-adsorbed peptide, the zigzag structure of the backbone of the PTFE-adsorbed peptide remains stable without being stretched and the bond angle and dihedral angle around each α-carbon are changed to form small angles. It is also envisaged that, if proline exists in a.PTFE-adsorbed peptide, the PTFE-adsorbed peptide is bent to form interactions with each of plural molecular chains of the PTFE.

According to the simulation results illustrated in FIGS. 12 and 15, on the other hand, it is envisaged that, if aromatic rings exist in side chains of a PTFE-adsorbed peptide, the zigzag structure of the backbone of the PTFE-adsorbed peptide, even without proline, remains stable without being stretched and the bond angle, and dihedral angle around each α-carbon are changed to form small angles.

According to the simulation results illustrated in FIG. 16, on the other hand, it is envisaged that, if the side chains of a PTFE-adsorbed peptide are short hydrophilic alanine residues, the zigzag structure of the backbone of the PTFE-adsorbed peptide is stretched and a strain is produced midway in the backbone of the PTFE-adsorbed peptide.

Based on the results of Table 2 and FIGS. 2 to 16, as amino acid residues constituting a PTFE-adsorbed peptide, relatively short amino acid residues having a polarity and an electron withdrawing property are considered to be effective in that their adsorbing forces to PTFE are high. Further, proline is considered to be effective in that it contributes to the stabilization of the structure of the backbone and the orientation of side chains of a PTFE-adsorbed peptide. For a PTFE-adsorbed peptide, it is considered to be preferable to have such a recurring structure as side chains are appropriately oriented.

Next, a description will be made regarding results of an adsorption experiment in which interactions between PTFE and PTFE-adsorbed peptides were measured.

FIG 17 is a diagram illustrating measurement results of contact angles of PTFE with the adsorptive peptides adsorbed thereon. FIG. 18 is a diagram illustrating results of dissociation of one of the adsorptive peptides from PTFE on which the adsorptive peptide was adsorbed.

FIG. 17 illustrates the measurement results of contact angles of droplets of ultrapure water when phage solutions were prepared through expression of the peptides by phage display and PTFE was immersed for 1 hour in the phage solutions. As the peptides, the PTFE-adsorbed peptides of SEQ ID NOs. 3-1 to 3-7 and the wild-type (WT) structure protein of M13 phage were used. FIG. 18 illustrates the results of quantification of dissociated amounts of peptides adsorbed on PTFE. After each peptide was expressed in a phage and adsorbed on PTFE, the phage solution was replaced with elution buffer, and the peptide was dissociated, eluted, and quantified.

In the PTFE-adsorbed peptides of SEQ ID NOs. 3-1 to 3-7, the contents of S, T, H, D, E, F, and N are 40% or higher. In the wild-type peptide, on the other hand, the content of S, T, H, D, E, F, and N is 30%. The amino acid sequence of the wild-type structure protein is AEGDDPAKAAFNSLQATEYIGYAWAMVVVIVGATIGIKLFKKFTSKAS.

As illustrated in FIG. 17, with the PTFE-adsorbed peptides of SEQ ID NOs. 3-1 to 3-7, the contact angles were smaller .compared with that of the wild-type peptide. The hydrophilicity of the surface of PTFE was presumably increased by the adsorption of the PTFE-adsorbed peptides of high polarity on PTFE. The wild-type peptide presumably did not sufficiently adsorb on PTFE for the relatively large value of the contact angle because the content of S, T, H, D, E, F, and N is as low as 30%.

As illustrated in FIG. 18, the PTFE-adsorbed peptide of SEQ ID NO. 3-1 in which the content of S, T, H, D, E, F, and N is 40% or higher was smaller in the associated amount of the peptide compared with the wild-type peptide in which the content of S, T, H, D, E, F, and N is lower. It is therefore indicated that a PTFE-adsorbed peptide, in which the content of S, T, H, D, E, F, and N is 40% or higher, is hard to dissociate from PTFE, on which the PTFE-adsorbed peptide is adsorbed, and has high affinity and adhesion with PTFE.

The PTFE-adsorbed peptide therefore preferably has an amino acid sequence, which contains at least one type of amino acid residue or residues among 2,3,4,5,6-pentafluorophenylalanine (Z), 3-(trifluoromethyl)alanine (X), serine (S), threonine (T), histidine (H), aspartic acid (D), glutamic acid (E), phenylalanine (F), or asparagine (N), and has a recurring structure.

The PTFE-adsorbed peptide may be formed using only Z, X, S, T, H, D, E, F, and N, or may be formed using one or more types of other amino acid residues. The amino acid residues that constitute the PTFE-adsorbed peptide are each of the L-type.

The PTFE-adsorbed peptide has separation energy of preferably 25.0 kJ/mol or higher, more preferably 30.0 kJ/mol or higher, still more preferably 34.0 kJ/mol or higher, even more preferably 35.0 kJ/mol or higher, yet more preferably 38.0 kJ/mol or higher, yet still more preferably 40.0 kJ/mol or higher, yet even more preferably 60.0 kJ/mol or higher.

The PTFE-adsorbed peptide has a length of preferably 5 or more amino acid residues, more preferably 8 or more amino acid residues, still more preferably 10 or more amino acid residues. On the other hand, 100 or fewer amino acid residues are preferred, 20 or fewer amino acid residues are more preferred, and 15 or fewer amino acid residues are still more preferred. The longer the PTFE-adsorbed peptide, the higher affinity can be obtained with the resin. On the other hand, the shorter the PTFE-adsorbed peptide, the harder intramolecular or intermolecular aggregations take place, the easier the alterations such as chemical modifications and clustering. Particularly preferably, the PTFE-adsorbed peptide has a length of 12 amino acid residues.

In the PMMA-adsorbed peptide, 2,3,4,5,6-pentafluoro-phenylalanine (Z), 3-(trifluoromethyl)alanine (X), serine (S), threonine (T), histidine (H), aspartic acid (D), glutamic acid (E), phenylalanine (F), or asparagine (N) account preferably for 40% or more of the amino acid residues. The content of Z, X, S, T, H, D, E, F, and N in the total length of the amino acid sequence is more preferably 50% or higher, still more preferably 60% or higher, even more preferably 70% or higher, yet more preferably 80% or higher, yet still more preferably 90% or higher. On the other hand, the content of Z, X, S, T, H, D, E, F, and N may be set at 100%, lower than 90%, or lower than 80%.

The PTFE-adsorbed peptide preferably has a recurring structure containing Z, X, S, T, H, D, E, F, or N, more preferably has a recurring structure containing Z, X, S, T, H, D, or E, still more preferably has a recurring structure containing Z, X, S, or T, particularly preferably has a recurring structure containing Z or X.

Z, X, S, T, H, D, E, and N can form relatively strong interactions with the resin as their side chains have a polarity. With an amino acid sequence containing Z, X, S, T, H, D, E, or N, a PTFE-adsorbed peptide that shows high affinity with PTFE can be obtained accordingly. With an amino acid sequence containing F, on the other hand, a PTFE-adsorbed peptide, in which the structure of the backbone and the orientation of the side chains are stable, may be obtained.

In the PTFE-adsorbed peptide, proline preferably accounts for 5% or more of the amino acid residues. The content of proline in the total length of the amino acid sequence may be set at 10% or higher, or 15% or higher. On the other hand, the content of proline may be set at lower than 20%, lower than 15%, or lower than 10%.

Proline has a ring structure so that the bond angle or dihedral angle of its peptide bond is restricted or fixed. Proline residues therefore exhibits an action to stabilize the structure of the backbone, and an action to bend the backbone. Interposition of proline may therefore provide a PTFE-adsorbed peptide with the structure of the backbone and the orientation of side chains being stabilized, or a PTFE-adsorbed peptide that forms interactions with plural respective molecular chains of PTFE.

The PMMA-adsorbed peptide is preferably a peptide containing an amino acid sequence represented by any one of the following SEQ ID NOs. (A-1) to (A-21) with one or two amino acid residues added thereto, inserted thereinto, substituted therefor, or deleted therefrom, more preferably a peptide containing an amino acid sequence represented by any one of the following SEQ ID NOs. (A-1) to (A-21), still more preferably a peptide containing two or more amino acid sequences represented by any one of the following SEQ ID NOs. (A-1) to (A-21) as recurring units:

ZZXXZ... (A-1)
ZXXZZ... (A-2)
XXZZX... (A-3)
XZZXX... (A-4)
STSTS... (A-5)
TSTST...(A-6)
SSSSS... (A-7)
TTTTT... (A-8)

HHHHH...(A-9)
DSDSD...(A - 10)
SDSDS... (A-11)
DHDHD... (A-12)
HDHDH... (A-13)
DEDED... (A-14)
EDEDE... (A-15)
FFHHF...(A-16)
FHHFF ... (A-17)
HHFFH ... (A-18)
HFFHH... (A-19)
NENEN... (A-20)
ENENE... (A-21)

where Z denotes a 2,3,4,5,6-pentafluorophenylalanine residue, and X represents a 3-(trifluoromethyl)alanine residue.

The PMMA-adsorbed peptide is more preferably a peptide containing an amino acid sequence represented by any one of the following SEQ ID NOs. (2-1) to (2-22) with one or two amino acid residues added thereto, inserted thereinto, substituted therefor, or deleted therefrom, still more preferably a peptide containing an amino acid sequence represented by any one of SEQ ID NOs. (2-1) to (2-22), particularly preferably an amino acid sequence represented by any one of SEQ ID NOs. (2-1) to (2-22):

ZZXXZZXXZZXX... (2-1)
ZXXZZXXZZXXZ... (2-2)
STSTSPSTSTST... (2-3)
TSTSTPTSTSTS... (2-4)
SSSSSPSSSSSS... (2-5)
TTTTTPTTTTTT... (2-6)
STSTSTSTSTST... (2-7)
TSTSTSTSTSTS... (2-8)
SSSSSSSSSSSS... (2-9)
TTTTTTTTTTTT... (2-10)
HHHHHHHHHHHH ... (2-11)
HHHHHPHHHHHH... (2-12)
DSDSDPDSDSDS... (2-13)
DSDSDSDSDSDS... (2-14)
DHDHDHDHDHDH...(2-15)
DHDHDPDHDHDH...(2-16)
DEDEDPDEDEDE... (2-17)
DEDEDEDEDEDE... (2-18)
FHHFFHHFFHHF...(2-19)
FFHHFFHHFFHH...(2-20)
NENENPNENENE... (2-21)
NENENENENENE... (2-22)

The PMMA-adsorbed peptide may also be a peptide containing an amino acid sequence that has an identity of 80% or greater with the amino acid sequence represented by any one of the following SEQ ID NOs. (3-1) to (3-7). The identity of the amino acid sequence is preferably 85% or greater, more preferably 90% or greater, still more preferably 95% or greater, particularly preferably 100%.

VHFPTKISEGDM... (3-1)
TFTLNSVHRSVH... (3-2)
SPHLHTSSPWER... (3-3)
FIESKTPVDPDG... (3-4)
GSESRTLFHPEG... (3-5)
EALTVNIKREME... (3-6)
SMIVEPRMLSTH... (3-7)

The PTFE-adsorbed peptide may also have one or more types of amino acid sequences having specific functions in addition to the amino acid sequence of any one of SEQ ID NOs. A-1 to A-21, SEQ ID NOs. 2-1 to 2-22, and SEQ ID NOs. 3-1 to 3-7. The PTFE-adsorbed peptide may have such an amino acid sequence on a side closest to the N-terminus among all the amino acid residues, or may have it at an intermediate position among all the amino acid sequences.
The PTFE-adsorbed peptide may also have one or a plurality of the amino acid sequences of SEQ ID NOs. A-1 to A-21, SEQ ID NOs. 2-1 to 2-22, and SEQ ID NOs. 3-1 to 3-7 in the total length. The PTFE-adsorbed peptide may have a single kind or plural types of these amino acid sequences.
The N-terminus and C-terminus of the PTFE-adsorbed peptide may be chemically modified, or may be in an optional ionization state. For example, the N-terminus may be any one of -NH2, $-NH_3^+$, $-CH_3CO$, 9-fluorenylmethyloxycarbonyl (Fmoc) group, tert-butoxycarbonyl (Boc) group, or the like. The C-terminus may be any one of -COOH, -COO-, $-CONH_2$, $-CONH_3^+$, or the like.
If the PTFE-adsorbed peptide is a peptide containing an amino acid sequence of any one of SEQ ID NOs. A-1 to A-21, SEQ ID NOs. 2-1 to 2-22, and SEQ ID NOs. 3-1 to 3-7 or an amino acid sequence similar to the above-mentioned amino acid sequence, the separation energy with a fluorinated resin has a higher theoretical value, so that high affinity is obtained to the fluorinated resin. Accordingly, the peptide adsorbed on the fluorinated resin hardly undergoes separation, thereby enabling to obtain a biocompatible material the biocompatibility of which lasts over a longer term.
Similar to the PMMA-adsorbed peptide, the PTFE-adsorbed peptide can be synthesized using a variety of synthesis methods including, for example, chemical synthesis methods such as liquid-phase synthesis methods and solid-phase synthesis methods, genetic engineering synthesis methods, and the like.

<Fluorinated Resin>

As the resin on which the PTFE-adsorbed peptide is to be adsorbed (the resin 1 that constitutes the biocompatible material 10), any optional resin can be used insofar as it is a fluorinated resin having fluorinated olefin units.
The resin on which the PTFE-adsorbed peptide is to be adsorbed may be a polymer of a fluorinated monomer, or a copolymer of a fluorinated monomer and another monomer. The copolymer may be any one of a block copolymer, a random copolymer, or a graft copolymer.
As the resin on which the PTFE-adsorbed peptide is to be adsorbed, polytetrafluoroethylene (PTFE) is particularly preferred. If the resin on which the PTFE-adsorbed peptide is to be adsorbed is PTFE, fluorine atoms as substituents are arranged in a stereoregular and uniform conformation, so that high affinity is available.
The fluorinated resin can be synthesized using a general synthesis method. If a fluorinated olefin such as tetrafluoroethylene is subjected to a radical polymerization, for example, a fluorinated resin is obtained in a powder form or the like. When melted, a fluorinated resin has high viscosity and low fluidity. When a fluorinated resin in a powder form or the like is allowed to cool after melting, however, a molded body, a non-molded solid body, or the like of the fluorinated resin is obtained. As a radical polymerization method, any one of emulsion polymerization, suspension polymerization, bulk polymerization, solution polymerization, or the like can also be used.

<Production Method of Biocompatible Material>

The biocompatible material 10 according to this embodiment can be produced, for example, by a method that brings a liquid in which the adsorptive peptide 2 is dispersed into contact with the resin 1 as a main part of the material. Usable as the liquid is a buffer with a variety of additives such as a pH regulator, a buffer agent, a salt, a reducing agent, and an organic solvent added therein, water, or the like.
As a method for bringing the liquid into contact with the resin 1, a variety of methods can be used including, for example, a method that coats the resin 1 with the liquid, a method that sprays the liquid onto the resin 1, a method that immerses the resin 1 in the liquid, and the like. When the liquid is caused to flow over the resin 1 for bringing the former into contact with the latter, control of the flow rate of the liquid at 20 $\mu$L/min or lower provides a higher adsorption efficiency for the adsorptive peptide 2.

<Combination of Biocompatible Material into Composite>

The biocompatible material 10 according to this embodiment can also be used in a form in which the resin 1 as a main part of the material is combined with a fibrous material or a filler material into a composite, a form in which the resin 1 is laminated on a surface of an adherend different from the resin 1, or a form in which the resin 1 constitutes a housing for a device.
FIG. 19 is a diagram schematically illustrating a biocompatible material as a composite combined with a fibrous material.
As illustrated in FIG. 19, a biocompatible material 10A combined with the fibrous material into the composite

includes a resin 1 as a main part of the material, an adsorptive peptide (peptide) 2 adsorbed on the resin 1, and a fibrous material 4 embedded in the resin 1.

In the biocompatible material 10A, the fibrous material 4 is embedded in a matrix of the resin 1. The fibrous material 4 may be dispersed in a form of short whiskers or the like in the matrix, or may be formed into a knitted, woven, or braided fabric, or the like and may then be embedded in the matrix. The fibrous material 4 can be combined into a composite by dispersing or otherwise incorporating it in the resin 1, for example, before polymerizing the resin 1 or before subjecting a preform of the powdery resin 1 to melt forming.

As the fibrous material 4, organic fibers, inorganic fibers, and metal fibers may each be used. Examples of the organic fibers include polyamide fibers, polyester fibers, aramid fibers, cellulose fibers, fluorinated resin fibers, and the like. Examples of the inorganic fibers include carbon fibers, silicon carbide fibers, glass fibers, boron fibers, alumina fibers, zirconia fibers, mullite fibers, rock wool, and the like. As the fibrous material 4, a single kind of such a fibrous material or a plurality of kinds of such fibrous materials may be used. Preferred as the fibrous material 4 to be combined with the fluorinated resin into a composite are inorganic fibers and/or metal fibers having a higher melting point than the fluorinated resin.

FIG. 20 is a diagram schematically illustrating another biocompatible material as a composite combined with a filler material.

As illustrated in FIG. 20, a biocompatible material 10B combined with the filler material into the composite includes the resin 1 as a main part of the material, the adsorptive peptide (peptide) 2 adsorbed on the resin 1, and a filler material 5 embedded in the resin 1.

In the biocompatible material 10B, the filler material 5 is embedded in a matrix of the resin 1. The filler material 5 may be in any shape such as a granular (spherical) shape, a flaky shape, or plate shape. Further, the filler material 5 may be in a solid form, or in a hollow form. The filler material 5 can be combined into a composite by dispersing it in the resin 1, for example, before polymerizing the resin 1 or before subjecting a preform of the powdery resin 1 to melt forming.

As the filler material 5, any one of an organic material, an inorganic material, or a metal material may be used. Examples of the organic material include polyamides, polyesters, aramids, fluorinated resins, and the like. Examples of the inorganic material include graphite, carbon black, silicon carbide, glass, silica, alumina, zirconia, mullite, mica, talc, kaolin, calcium carbonate, magnesium carbonate, aluminum hydroxide, titanium oxide, iron oxide, ferrite, and the like. As the filler material 5, a single kind of such a filler material or a plurality of kinds of such filler materials may be used. Preferred as the filler material 5 to be combined with the fluorinated resin into a composite is an inorganic material and/or a metal material having a higher melting point than the fluorinated resin.

With the biocompatible material 10A illustrated in FIG. 19 or the biocompatible material 10B illustrated in FIG. 20, a variety of advantages such as improved stiffness, improved dielectric characteristics, and improved pressure resistance performance can be obtained by the fibrous material 4 or the filler material 5 in addition to improvements in biocompatibility by effects of the adsorptive peptide 2. Accordingly, the biocompatibility lasts for a long term, thereby enabling to obtain a biocompatible material improved in the stiffness of the material itself and in performance according to a use.

FIG. 21 is a diagram schematically illustrating a further biocompatible material laminated on a surface of an adherend.

As illustrated in FIG. 21, the biocompatible material 10 laminated on the surface of the adherend 20 forms a composite material 100 of a multilayered structure, which is made up with the resin 1, the adsorptive peptide (peptide) 2 adsorbed on the resin 1, and the adherend 20 different from the resin 1.

The composite material 100 can be used as a material for optional articles which have a possibility of contact with a living body. The articles formed with the composite material 100 are not particularly limited in kind, use, function, and so on insofar as they can be brought into contact with living bodies. Similar to the above-described biocompatible material 10 itself, the composite material 100 can form the articles exemplified as specific examples.

In the composite material 100, the adsorptive peptide 2 is adsorbed on a front side of the biocompatible material 10, and the adherend 20 is joined to a back side of the biocompatible material 10 where the adsorptive peptide 2 is not adsorbed. The joining of the biocompatible material 10 and the adherend 20 can be conducted by an appropriate method, such as a method that polymerizes the resin 1 on a surface of the adherend 20, compression bonding, welding, or adhesion, according to the kind of the adherend 20.

The adherend 20 is a material different from the resin 1, and constitutes the main part of the composite material 100. The adherend 20 can be formed into an appropriate shape according to the kind, use, function and the like of the article to be formed with the composite material 100, and is not particularly limited in shape. The adherend 20 may be covered with resin 1 in the shape of a film or sheet to constitute the composite material 100, or may be bonded with the resin 1 formed in a similar shape as the adherend 20 to constitute the composite material 100. The adherend 20 and the resin 1 may constitute the entire area or only a part of the article.

As the adherend 20, any of organic materials such as natural polymers, synthetic polymers, and foamed resins, inorganic materials such as alumina, zirconia, and glass, and metal materials such as stainless steel, aluminum alloys,

copper alloys, cobalt alloys, and titanium alloys may be used. The adherend 20 may have biocompatibility by itself, or may have no biocompatibility by itself. If the adherend 20 itself has biocompatibility, the adherend 20 may be covered at a part or the entire area of its surface with the biocompatible material 10.

With the biocompatible material 10 illustrated in FIG. 21, the composite material 100 provided with biocompatibility can be formed using the adherend 20 having desired properties different from those of the resin with methoxycarbonyl groups and methyl groups contained therein or the fluorinated resin. The adherend 20 is not required to have affinity with the adsorptive peptide 2 or biocompatibility. Therefore, the adherend 20 is high in the degree of freedom in its selection, and can bring about a variety of advantages such as improved stiffness, improved dielectric characteristics, improved pressure resistance performance, and reduced cost. It is hence possible to obtain, with no much restrictions, a composite material provided with long-lasting biocompatibility and performance according to its use.

FIG. 22 is a diagram schematically illustrating a still further biocompatible material that constitutes a housing for a device.

As illustrated in FIG. 22, the biocompatible material 10 which constitutes the housing for the device 30 forms an implantable device 200 by covering the surface of the device 30 in the housing made of the resin 1 and the adsorptive peptide (peptide) 2 adsorbed on the resin 1.

The implantable device 200 is implanted in a living body, and operates for a variety of purposes. No particular limitation is imposed on the kind, use, function, and the like of the implantable device 200. As a specific example of the implantable device 200, there may be given such an artificial heart described in Non-Patent Document (Tomoko Sugiyama et al., "Evaluation of Biocompatibility of Long-term Implantable Device Material in Subcutaneous Implantation Experiment," JINKO ZOKI (in Japanese), Japan Society for Artificial Organs, April 15th, 1998, 28(2), p. 509 to 513). In addition, artificial pancreases, artificial organs such as pacemakers, artificial joints, artificial tissues such as artificial muscles, electronic devices such as IC (integrated circuit) tags and IC chips, and the like are exemplified.

In the implantable device 200, the adsorptive peptide 2 is adsorbed on a surface of the resin 1 that constitutes the housing, and the device 30 is accommodated on back sides of the resin 1 where the adsorptive peptide 2 is not adsorbed. The device 30 can be joined to the resin 1 by an appropriate method such as compression bonding, welding, adhesion, or mechanical joining that uses fasteners or the like, or can be covered with the resin 1 without joining it to the resin 1.

The device 30 is formed of at least one of various system components, electronic components, and the like, each of which uses an artificial material or the like such that the device 30 operates for a variety of purposes such as a treatment, a replacement for a biofunction, an aid to a biofunction, recording of information, and calculation of information. The device 30 can be formed into an appropriate shape according to the kind, use, function, and the like of the implantable device 200, and no particular limitation is imposed on its shape. The device 30 may be covered at a part or the entire area of its surface with the biocompatible material 10. Further, the device 30 may autonomously operate in the body, or may operate with equipment outside the body connected thereto via a tubing, a control line, or the like.

With the biocompatible material 10 illustrated in FIG. 22, the implantable device 200 which gives little adverse effect or irritation to a living body can be formed by covering the device 30 which operates for various purposes in the housing that shows biocompatibility. The housing that accommodates the device 30 therein can be molded with a high degree of freedom from the resin 1, and irrespective its shape, can be efficiently imparted with biocompatibility by the adsorptive peptide 2 through simple processing or operation. It is therefore possible to efficiently manufacture an implantable device which has long-lasting biocompatibility and fulfils a desired function in the body.

<Functional Material>

Through combinations with various functional substances into composites, the biocompatible material 10 according to this embodiment can also be formed into functional materials that fulfil particular functions by themselves.

FIGS. 23, 24, and 25 are diagrams schematically illustrating configuration examples of functional materials.

As illustrated in FIGS. 23, 24, and 25, the functional materials 300 (300A, 300B, and 300C) according to this embodiment each include the resin 1 as a main part of the material, the adsorptive peptide (peptide) 2 adsorbed on the resin 1, and a functional substance 6 that functions inside a living body or on the surface of a living body.

As the functional substance 6, a variety of substances that fulfil specific functions through biological effects, chemical effects, or the like in living bodies or on the surfaces of living bodies can be used. The functional substance 6 may be adsorbed on the resin 1, or may be adsorbed on the adsorptive peptide 2 adsorbed on the resin 1. The adsorptive peptide 2 can have a function to have the functional substance 6 which is to adsorb to a front side of the functional material 300, adhered or bonded, in addition to the function to improve the biocompatibility of the functional material 300 on the front side thereof.

As the functional substance 6, a variety of substances such as physiologically active substances and antibacterial substances can be used, for example. During use of the functional material 300, the functional substance 6 may function while being held on the surface of the biocompatible material 10, or may function while being released from the

surface of the biocompatible material 10. The functional substance 6 may be adsorbed by physical adsorption, or may be adsorbed by chemical adsorption.

Specific examples of physiological substances include inflammation suppressing proteins such as interleukin-10, and angiogenesis promoting proteins such as interleukin-8. Further, other interleukins, interferons, cytokines such as chemokines, hormones, and the like are also exemplified. In addition, antibiotics, anticoagulants, antihistamines, nutrients such as vitamins, sedatives, analgesics, anti-inflammatories, steroids, insulin, and the like are also exemplified.

Specific examples of antibacterial substances include metal particles of silver, copper, zinc, titanium, zirconium, cobalt, nickel, and the like, inorganic compounds of these metals, metal supports with these metals carried on a support such as zeolite, and the like.

As illustrated in FIG. 23, the functional material 300 can have a configuration (the functional material 300A) that the adsorptive peptide 2 is adsorbed on parts of the surface of resin 1 and the remaining parts of the surface of the resin 1 and the adsorptive peptide 2 are covered with the functional substance 6. The functional substance 6 may be physically adsorbed on the adsorptive peptide 2, may be chemically adsorbed on the adsorptive peptide 2, or may be physically adsorbed on both the resin 1 and the adsorptive peptide 2.

As illustrated in FIG. 24, the functional material 300 can also have a configuration (the functional material 300B) that the adsorptive peptide 2 adsorbed on the entire area of the surface of the resin 1 is covered with the functional substance 6. The functional substance 6 may be physically adsorbed on the adsorptive peptide 2, or may be chemically adsorbed on the adsorptive peptide 2.

According to the functional materials 300A and 300B as described above, good biocompatibility is obtained from the adsorptive peptide 2 adsorbed on the surface of the resin 1. Especially if the functional substance 6 is released from the front side of the biocompatible material 10, the surface of the resin 1 is hardly exposed, thereby enabling to suppress adverse effect and irritation to a living body by the resin 1.

As illustrated in FIG. 25, the functional material 300 can also have a configuration (the functional material 300C) that parts of the adsorptive peptide 2 adsorbed on the entire area of the surface of the resin 1 is covered with the functional substance 6 and the adsorptive peptide 2 is exposed to a surface. The functional substance 6 may be physically adsorbed on the adsorptive peptide 2, or may be chemically adsorbed on the adsorptive peptide 2.

According to the functional material 300C as described above, good biocompatibility is obtained from the adsorptive peptide 2 exposed to the surface, irrespective of properties of the functional substance 6. Especially if the functional substance 6 is released from the front side of the biocompatible material 10, the surface of the resin 1 is not exposed, thereby enabling to prevent adverse effect and irritation to a living body by the resin 1.

The present invention has been described above. However, the present invention should not be limited to the above-described embodiment and modifications, and a variety of modifications is feasible within a range not departing from the spirit of the present invention. For example, the present invention is not necessarily limited to that including all the elements included in the above-described embodiment and modifications. One or some of the elements of any one of the embodiment and modifications can be replaced by other element or elements, one or some of the elements of any one of the embodiment and modifications can be added to other configuration or configurations, or one or some of the elements of any one of the embodiment and modifications can be omitted.

For example, the above-described fibrous material 4 or filler material 5 can be combined into a composite with the biocompatible material 10 which covers the adherend 20, or the biocompatible material 10 which constitutes the housing for the device 30. Further, the above-described adherend 20 can also be arranged so as to come into contact with the surface of the biocompatible material 10, the surface being on the side where the adsorptive peptide 2 is adsorbed. Furthermore, the above-described housing for the device 30 can also be formed with the biocompatible material 10 laminated on the adherend 20 such as titanium.

The above-described functional material 300 is not particularly limited in the state of adsorption, the shape, the structure, or the like insofar as it includes the resin 1, the adsorptive peptide 2 adsorbed on the resin 1, and the functional substance 6. The functional substance 6 itself may have an amino acid sequence that adsorbs to the resin 1, may be adsorbed on the adsorptive peptide 2, or may be bound to the adsorptive peptide 2 via covalent bonds or the like.

Reference Signs List

1: Resin
2: Adsorptive peptide
4: Fibrous material
5: Filler material
6: Functional substance
10: Biocompatible material

20: Adherend
30: Device
100: Composite material
200: Implantable device
300: Functional material

## Claims

1. A biocompatible material comprising:

   a resin; and
   a peptide adsorbed on the resin, wherein
   the resin has methoxycarbonyl groups and methyl groups, and
   tryptophan or arginine accounts for 70% or more of amino acid residues in the peptide.

2. The biocompatible material according to claim 1, wherein
   proline or glutamic acid accounts for 10% or more of the amino acid residues in the peptide.

3. The biocompatible material according to claim 1, wherein

the peptide contains a tryptophan residue as a 2n-th (where n represents a natural number, and the same will apply hereinafter) amino acid residue, an arginine residue as a (2n+2)th amino acid residue, and a tryptophan residue as a (2n+4)th amino acid residue, all, as counted from an N-terminus.

**4.** The biocompatible material according to claim 3, wherein the peptide contains

an arginine residue as a (2m-1)th (where m represents a natural number, and the same will apply hereinafter) amino acid residue, a tryptophan residue as a (2m+1)th amino acid residue, and a proline residue as a (2m+3)th amino acid residue, all, as counted from the N-terminus,
an arginine residue as a (2m-1)th amino acid residue, a tryptophan residue as a (2m+1)th amino acid residue, and an arginine residue as a (2m+3)th amino acid residue, all, as counted from the N-terminus,
a tryptophan residue as a (2m-1)th amino acid residue, a tryptophan residue as a (2m+1) th amino acid residue, and a proline residue as a (2m+3)th amino acid residue, all, as counted from the N-terminus, or
a glutamic acid residue as a (2m-1)th amino acid residue, a tryptophan residue as a (2m+1)th amino acid residue, and a proline residue as a (2m+3)th amino acid residue, all, as counted from the N-terminus.

**5.** The biocompatible material according to claim 1, wherein
the peptide is a peptide including an amino acid sequence with one or two amino acid residues added to, inserted into, substituted for, or deleted from an amino acid sequence represented by any one of the following SEQ ID NOs. (1-1) to (1-5):

RWWRPWW... (1-1)
RWWRRWW... (1-2)
WWWRPWW...(1-3)
EWWRPWR...(1-4)
RWWRPWR...(1-5)

**6.** The biocompatible material according to claim 1, wherein
the resin is poly(methyl methacrylate) resin.

**7.** The biocompatible material according to claim 1, wherein
the resin is isotactic poly(methyl methacrylate) resin.

**8.** A biocompatible material comprising:

a resin; and
a peptide adsorbed on the resin, wherein
the resin is a fluorinated resin, and
2,3,4,5,6-pentafluorophenylalanine, 3-(trifluoromethyl)alanine, serine, threonine, histidine, aspartic acid, glutamic acid, phenylalanine, or asparagine accounts for 40% or more of amino acid residues in the peptide.

**9.** The biocompatible material according to claim 8, wherein
the peptide is a peptide including an amino acid sequence represented by any one of the following SEQ ID NOs. (A-1) to (A-21) where Z denotes a 2,3,4,5,6-pentafluorophenylalanine residue, and X denotes a 3-(trifluoromethyl)alanine residue, or a peptide including an amino acid sequence with one or two amino acid residues added to, inserted into, substituted for, or deleted from an amino acid sequence represented by any one of the following SEQ ID NOs. (A-1) to (A-21):

ZZXXZ... (A-1)
ZXXZZ... (A-2)
XXZZX... (A-3)
XZZXX... (A-4)
STSTS...(A-5)
TSTST... (A-6)
SSSSS... (A-7)
TTTTT... (A-8)
HHHHH... (A-9)
DSDSD... (A-10)

SDSDS... (A-11)
DHDHD ... (A-12)
HDHDH... (A-13)
DEDED... (A-14)
EDEDE... (A-15)
FFHHF... (A-16)
FHHFF...(A-17)
HHFFH... (A-18)
HFFHH ... (A-19)
NENEN... (A-20)
ENENE... (A-21)

**10.** The biocompatible material according to claim 8, . wherein
the peptide is a peptide including an amino acid sequence represented by any one of the following SEQ ID NOs. (3-1) to (3-5), or a peptide including an amino acid sequence having an identity of 80% or greater to any one of the following SEQ ID NOs. (3-1) to (3-5):

VHFPTKISEGDM... (3-1)
SPHLHTSSPWER... (3-2)
FIESKTPVDPDG... (3-3)
EALTVNIKREME... (3-4)
SMIVEPRMLSTH... (3-5)

**11.** The biocompatible material according to claim 8, wherein
the resin is polytetrafluoroethylene.

**12.** The biocompatible material according to any one of claims 1 to 11, wherein
the resin is a composite with a fibrous material or a filler material.

**13.** The biocompatible material according to any one of claims 1 to 11, wherein
the resin is laminated on an adherend different from the resin.

**14.** The biocompatible material according to any one of claims 1 to 11, wherein
the resin constitutes a housing for a device including a system component or an electronic component.

**15.** A functional material comprising: the biocompatible material according to any one of claims 1 to 11; and

a functional substance that functions in a living body or on a surface of the living body, wherein
the functional substance is held on a surface of the biocompatible material, or is released from the surface of the biocompatible material.

FIG.1

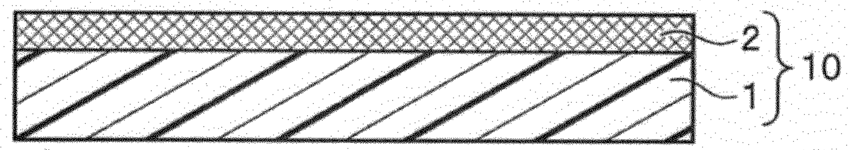
2
10
1

FIG.2

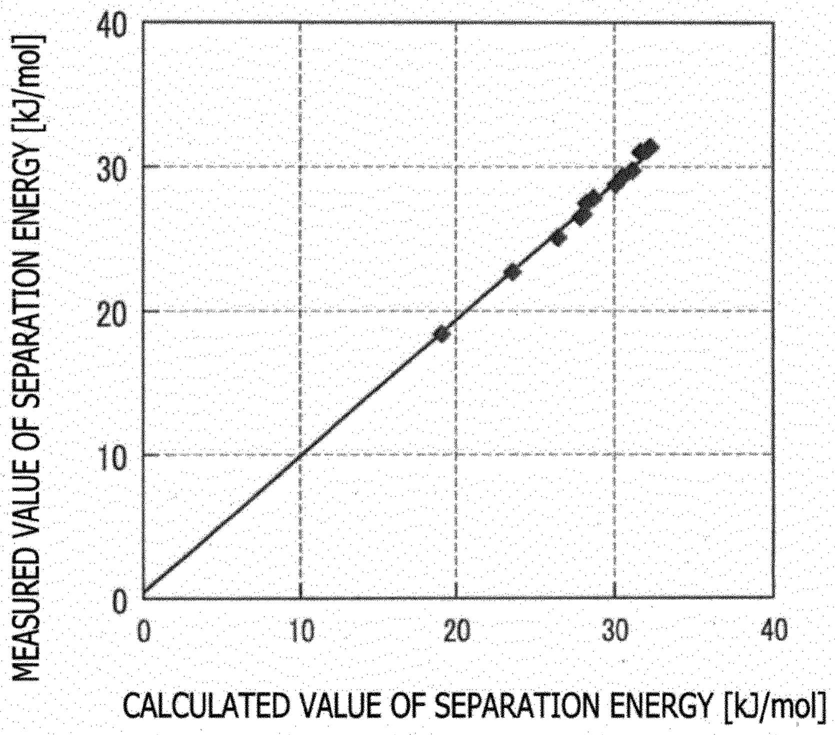
40
30
20
10
0
0
10
20
30
40
MEASURED VALUE OF SEPARATION ENERGY [kJ/mol]
CALCULATED VALUE OF SEPARATION ENERGY [kJ/mol]

FIG.3

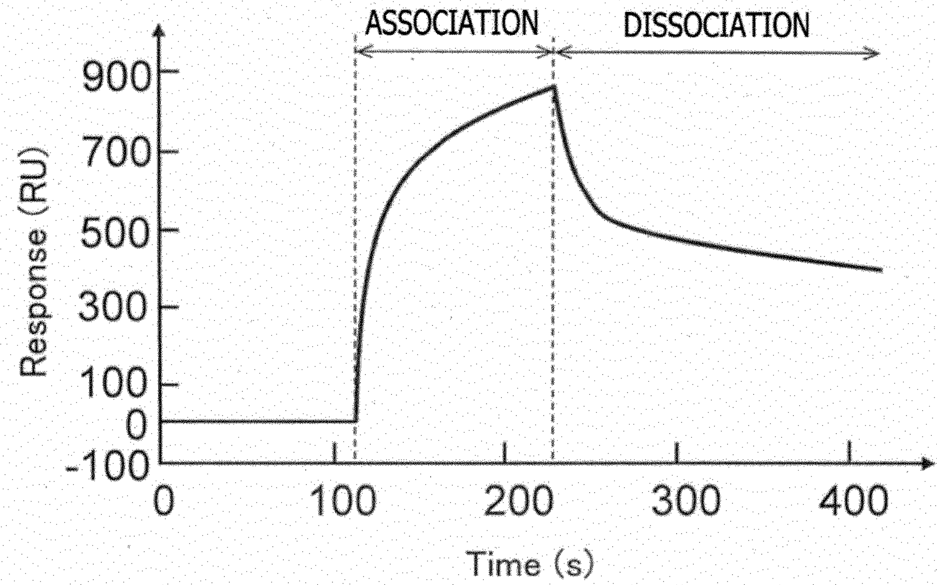
ASSOCIATION
DISSOCIATION
900
700
500
300
100
0
-100
Response (RU)
0
100
200
300
400
Time (s)

FIG.4

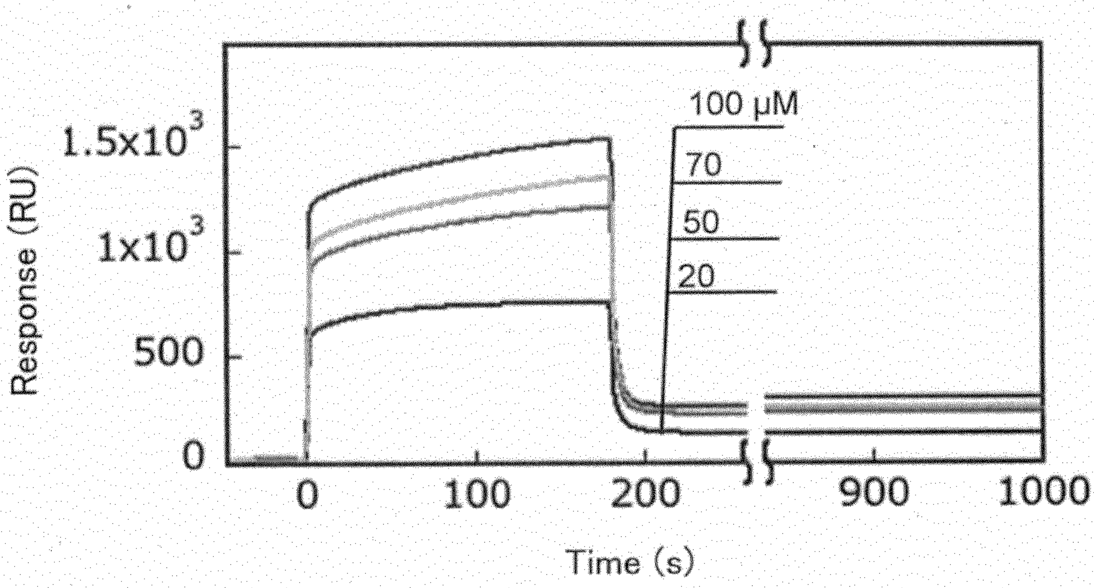
100 μM
70
50
20
1.5x10³
1x10³
500
0
Response (RU)
0
100
200
900
1000
Time (s)

FIG. 5

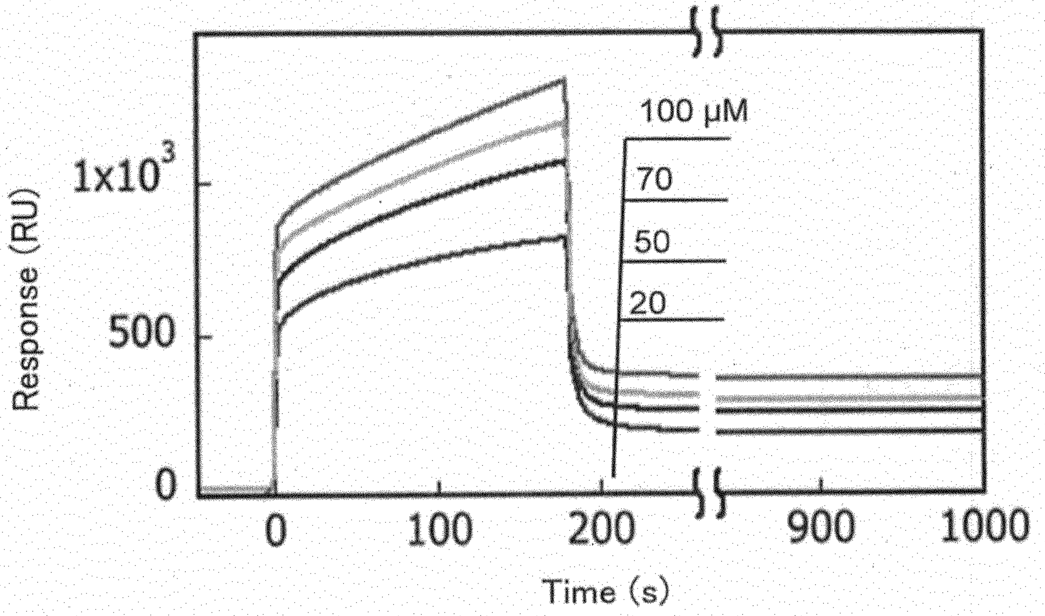
1x10^3
500
0
Response (RU)
100 µM
70
50
20
0
100
200
900
1000
Time (s)

FIG. 6

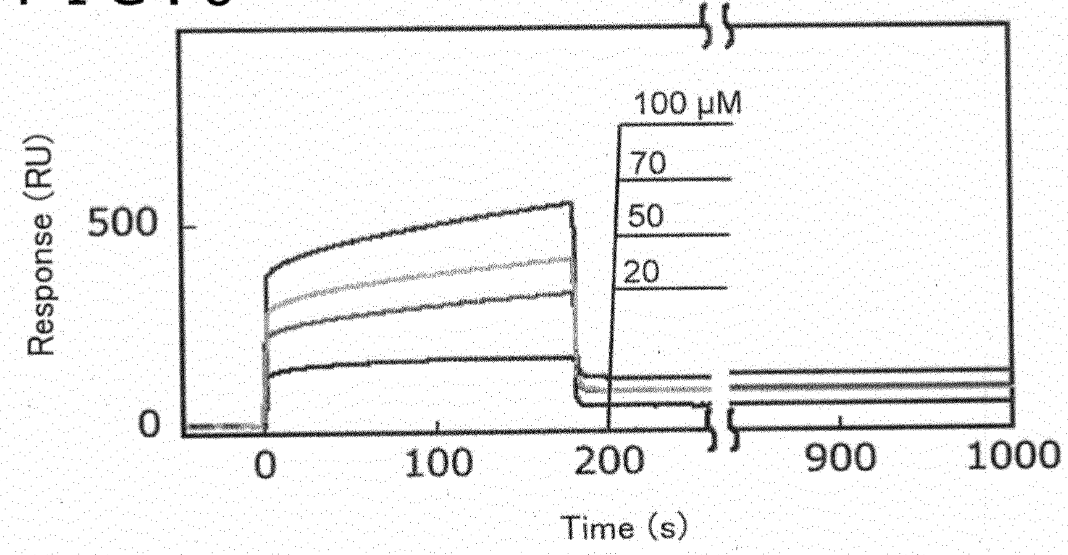
500
0
Response (RU)
100 µM
70
50
20
0
100
200
900
1000
Time (s)

FIG. 7

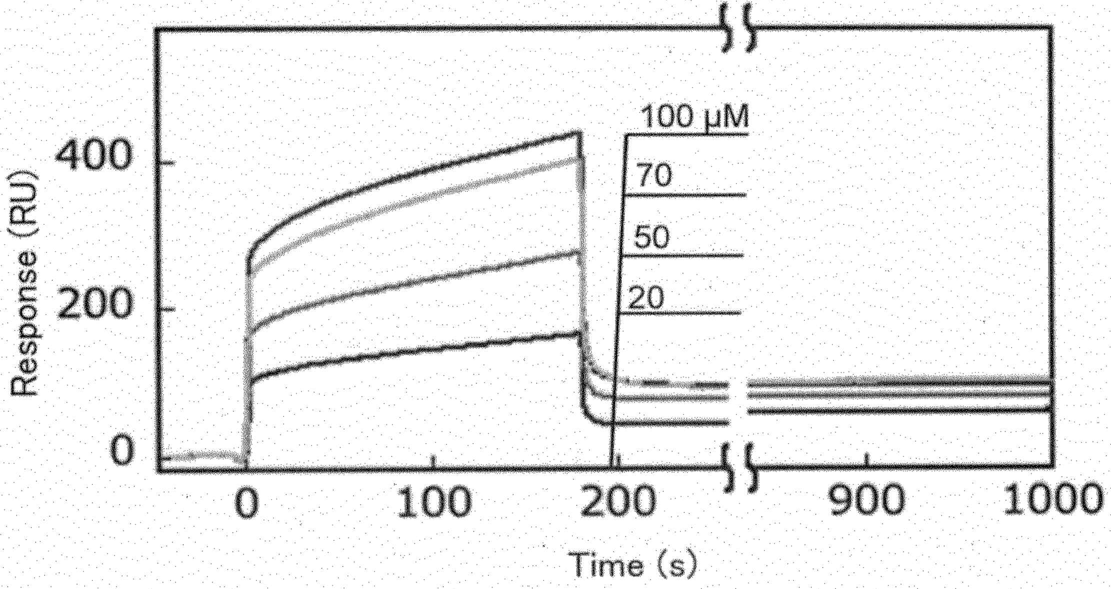

FIG. 8

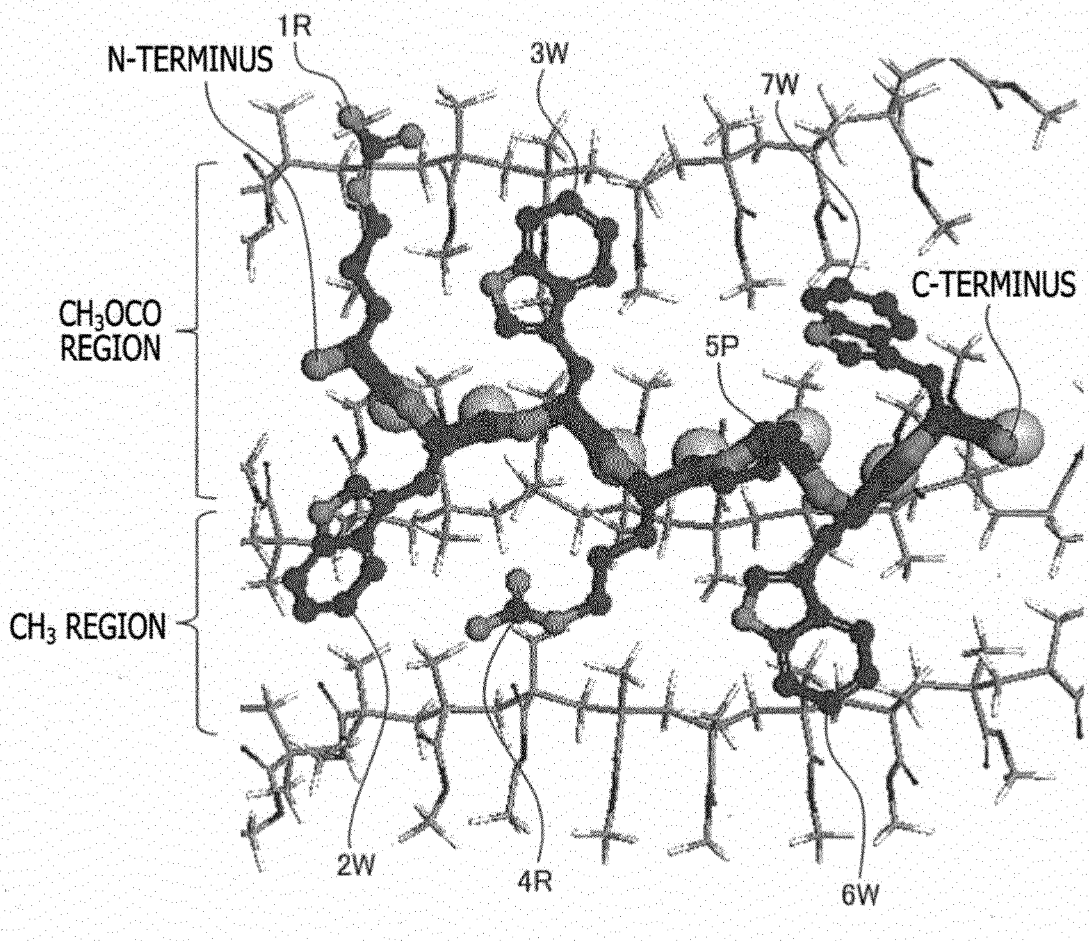
1R
N-TERMINUS
3W
7W
C-TERMINUS
CH3OCO REGION
5P
CH3 REGION
2W
4R
6W

FIG.9

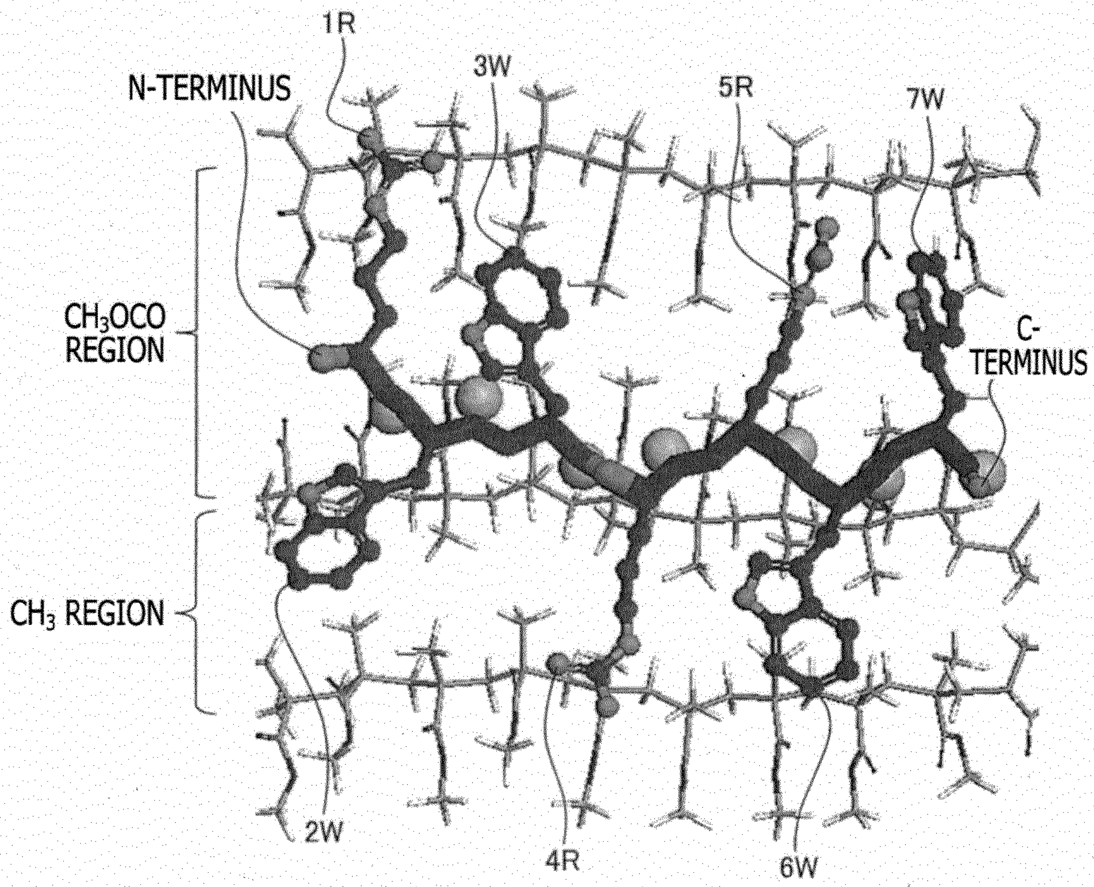
1R
N-TERMINUS
3W
5R
7W
$CH_3OCO$ REGION
C-TERMINUS
$CH_3$ REGION
2W
4R
6W

FIG. 10

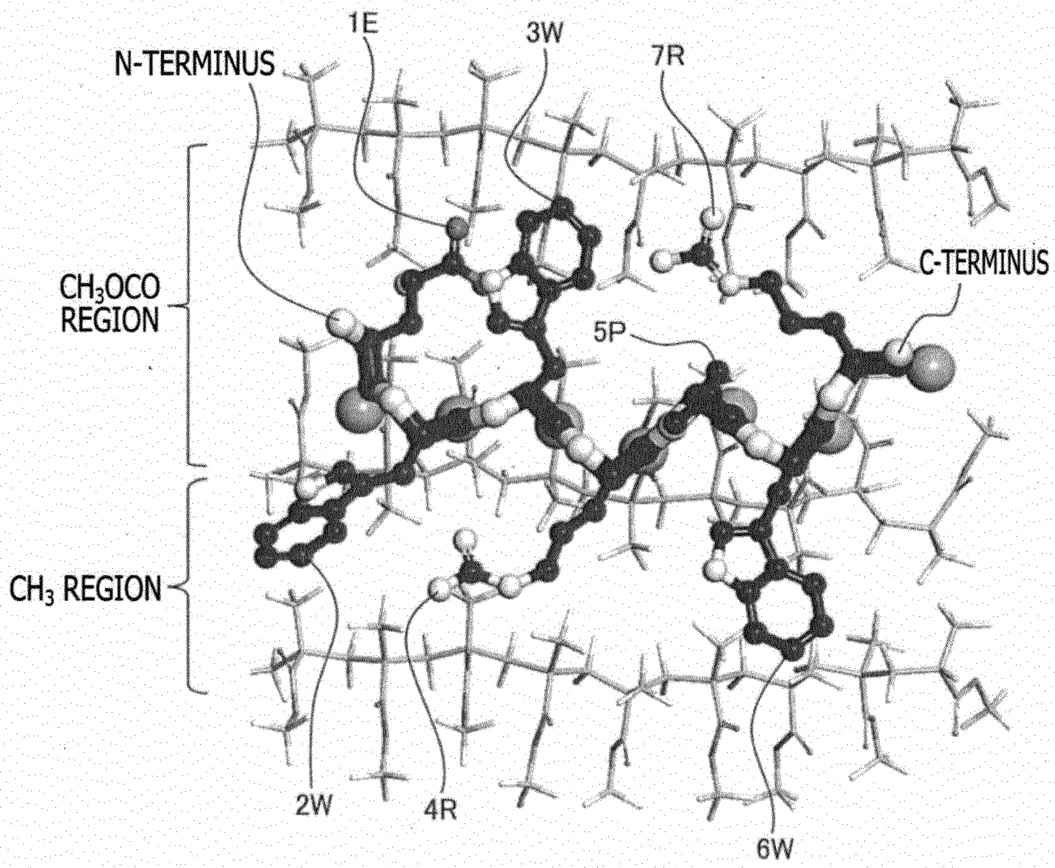
1E
3W
N-TERMINUS
7R
$CH_3OCO$ REGION
C-TERMINUS
5P
$CH_3$ REGION
2W
4R
6W

FIG.11

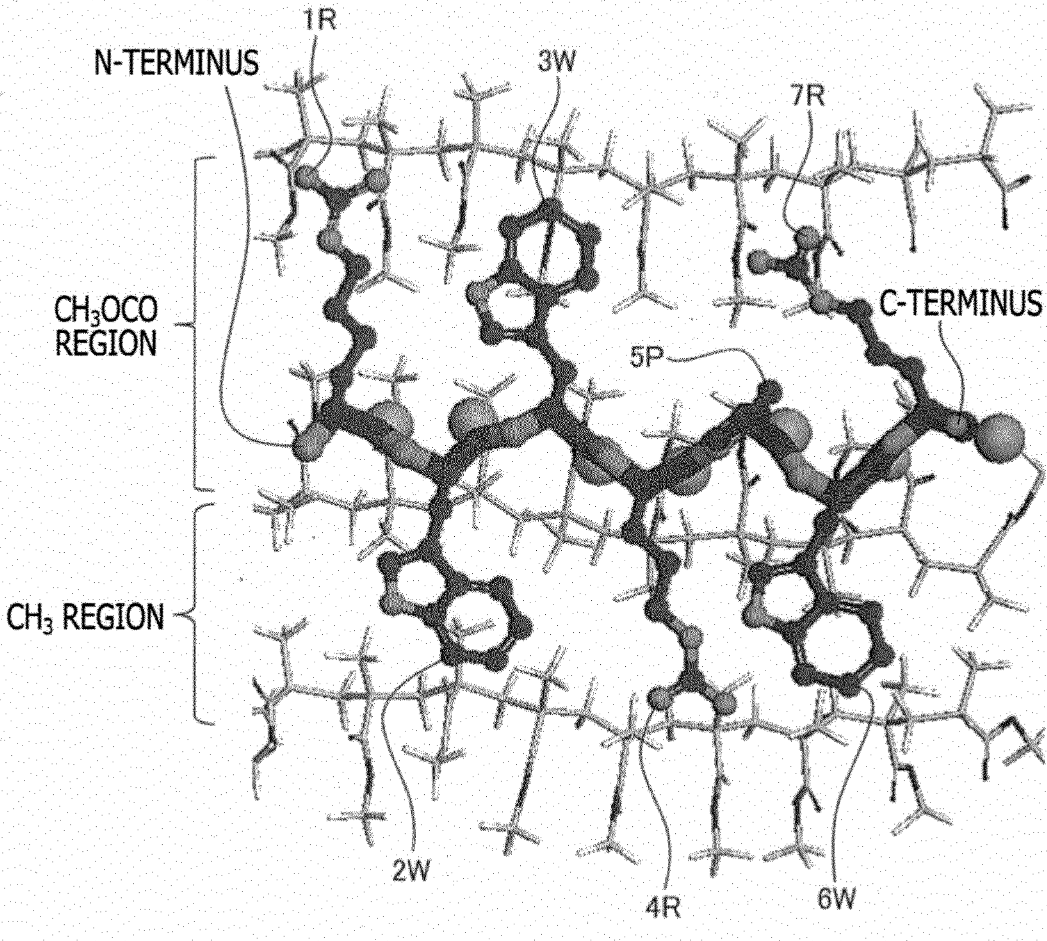
1R
N-TERMINUS
3W
7R
$CH_3OCO$ REGION
C-TERMINUS
5P
$CH_3$ REGION
2W
4R
6W

FIG. 12

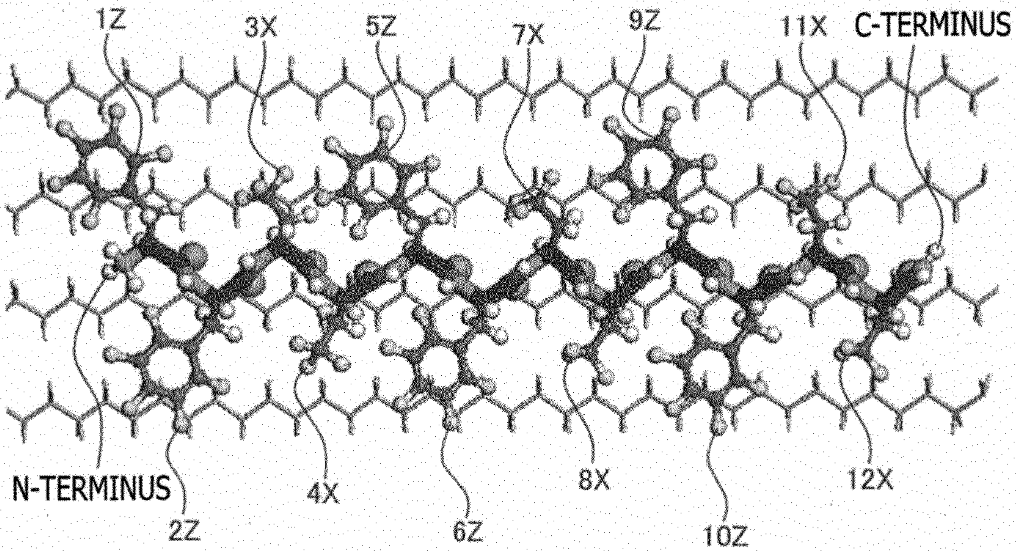

# FIG.13

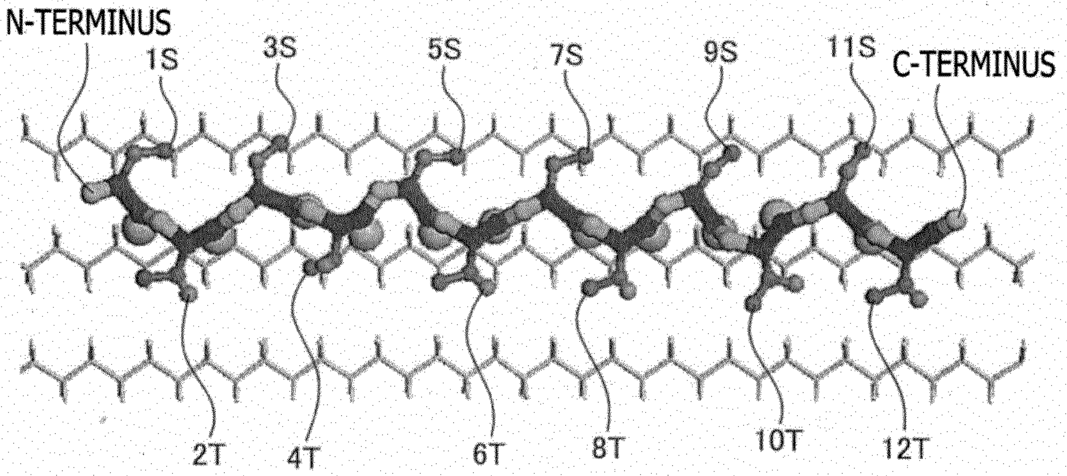
N-TERMINUS
1S
3S
5S
7S
9S
11S
C-TERMINUS
2T
4T
6T
8T
10T
12T

# FIG.14

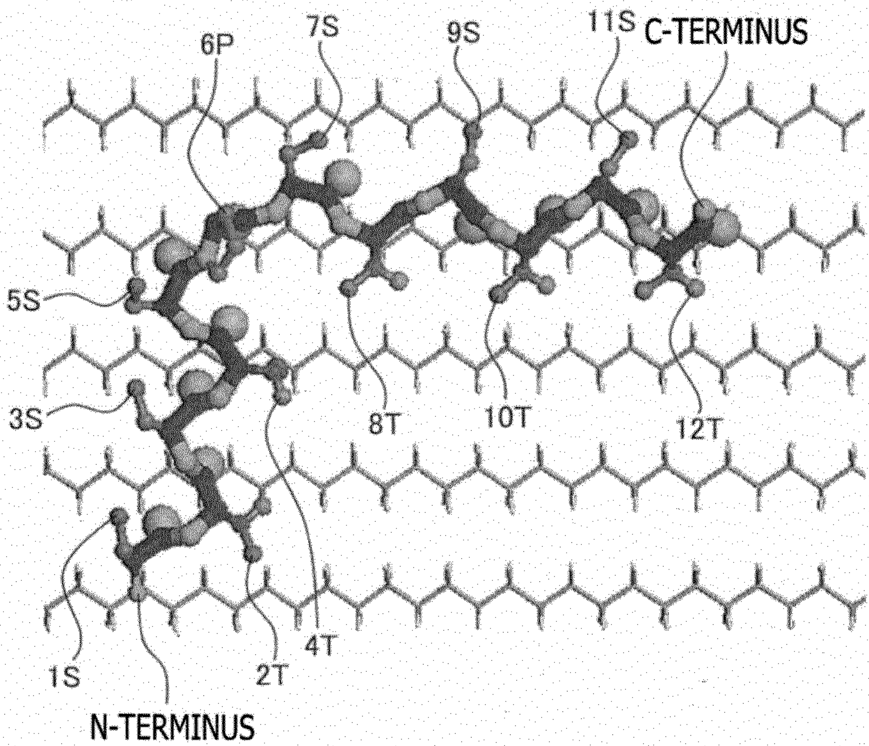
6P
7S
9S
11S
C-TERMINUS
5S
3S
8T
10T
12T
1S
2T
4T
N-TERMINUS

FIG. 15

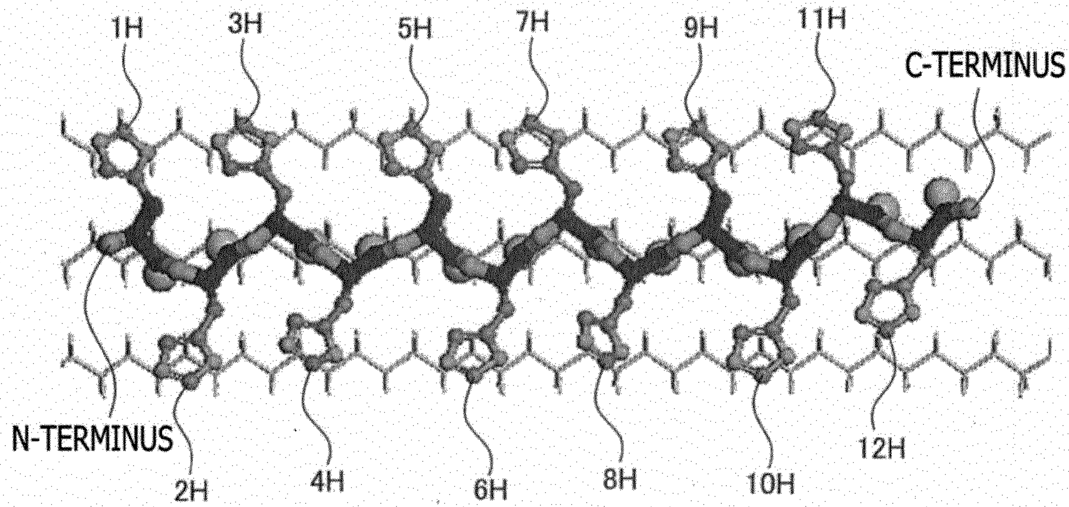
1H
3H
5H
7H
9H
11H
C-TERMINUS
N-TERMINUS
2H
4H
6H
8H
10H
12H

FIG. 16

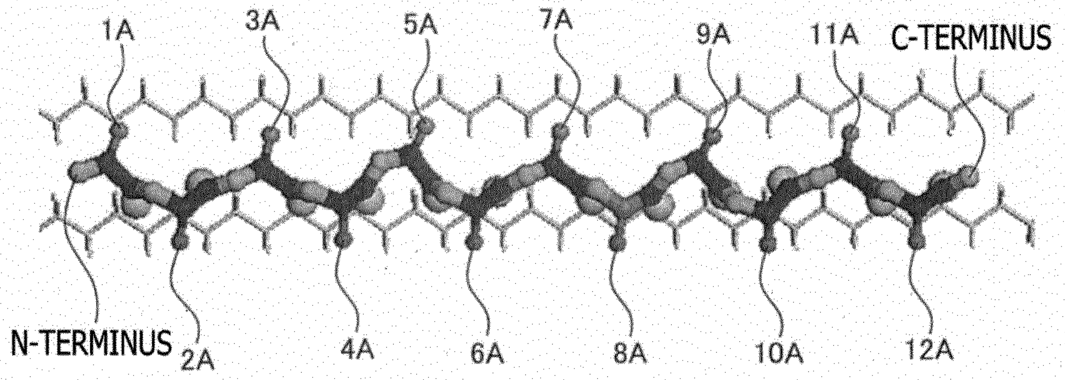
1A
3A
5A
7A
9A
11A
C-TERMINUS
N-TERMINUS
2A
4A
6A
8A
10A
12A

FIG. 17

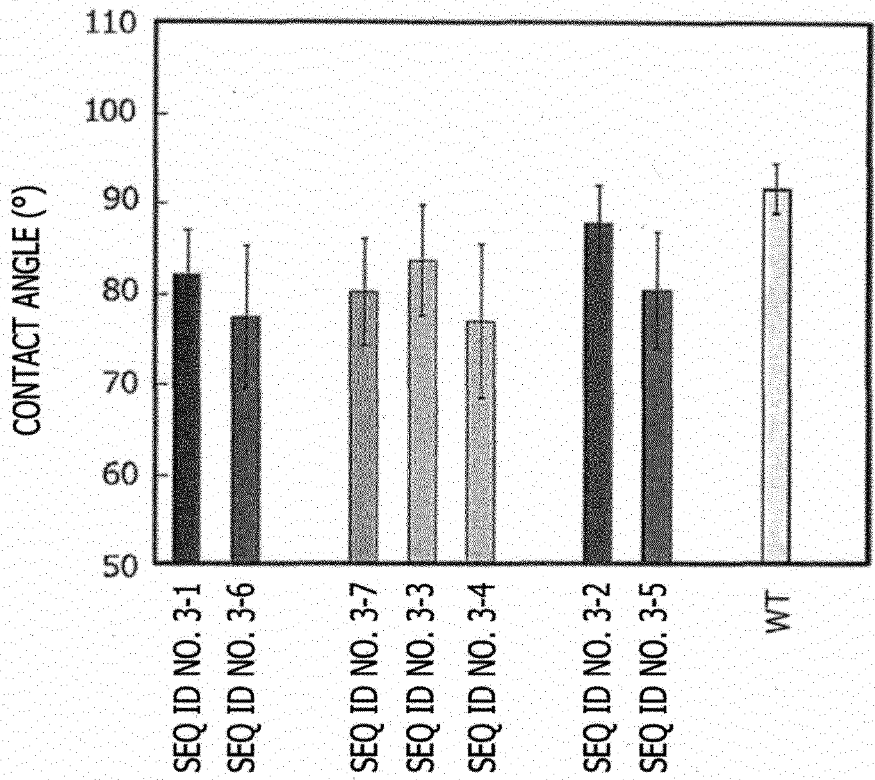
CONTACT ANGLE (°)
110
100
90
80
70
60
50
SEQ ID NO. 3-1
SEQ ID NO. 3-6
SEQ ID NO. 3-7
SEQ ID NO. 3-3
SEQ ID NO. 3-4
SEQ ID NO. 3-2
SEQ ID NO. 3-5
WT

FIG. 18

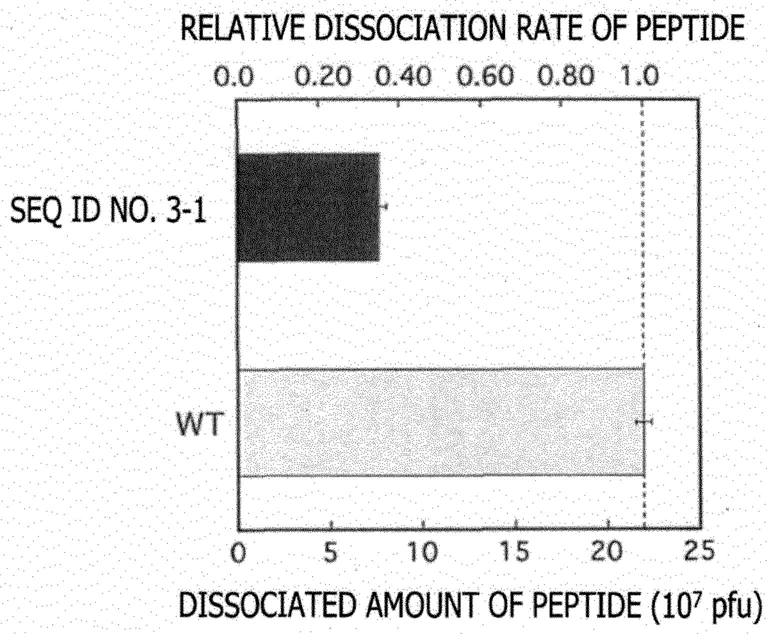
RELATIVE DISSOCIATION RATE OF PEPTIDE
0.0 0.20 0.40 0.60 0.80 1.0
SEQ ID NO. 3-1
WT
0 5 10 15 20 25
DISSOCIATED AMOUNT OF PEPTIDE (10⁷ pfu)

FIG.19

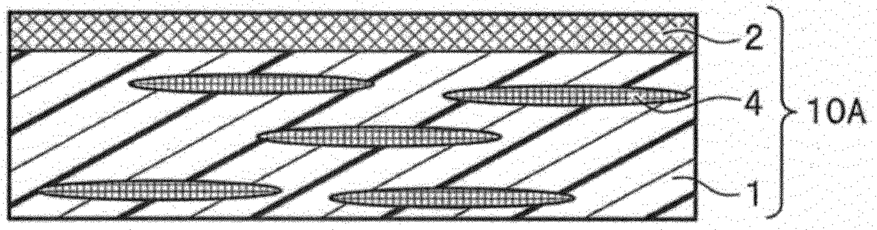


FIG.20

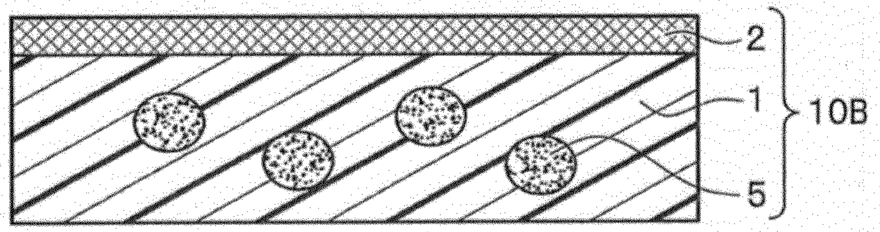

FIG.21

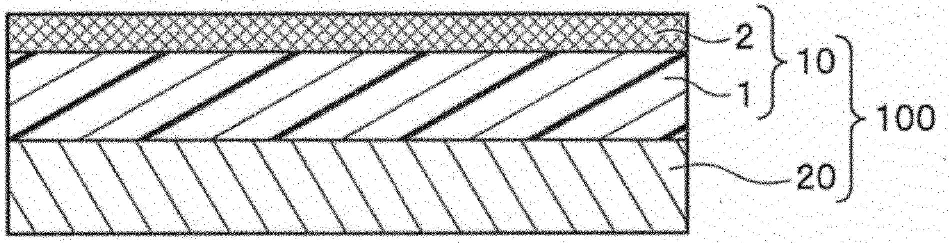


FIG.22

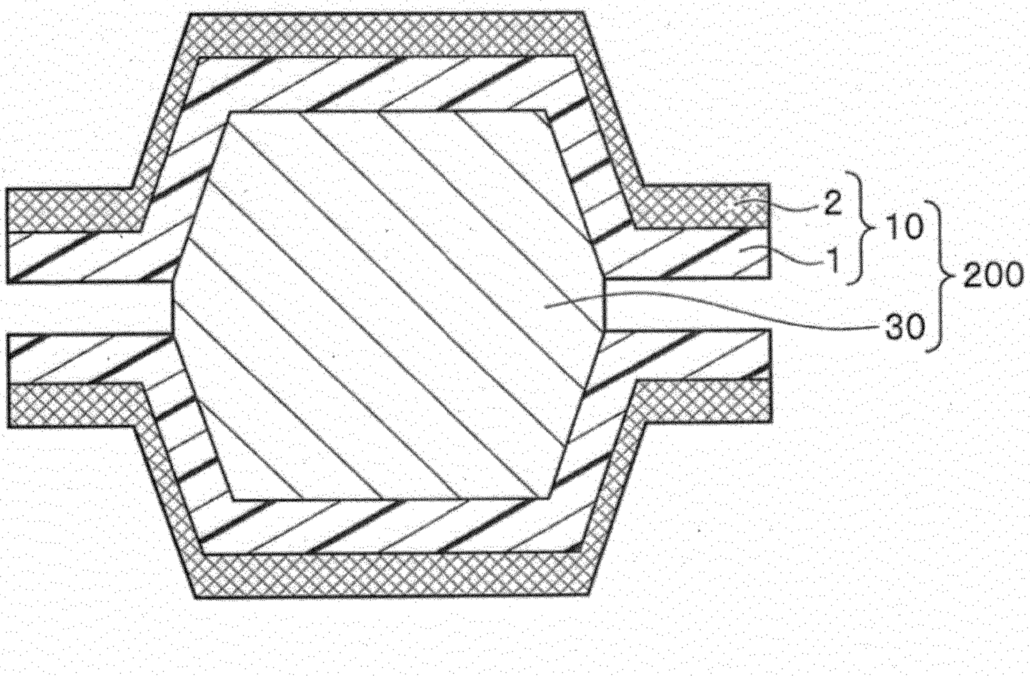

FIG.23

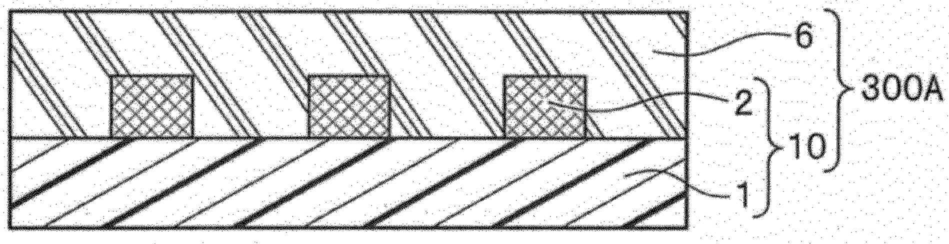


FIG.24

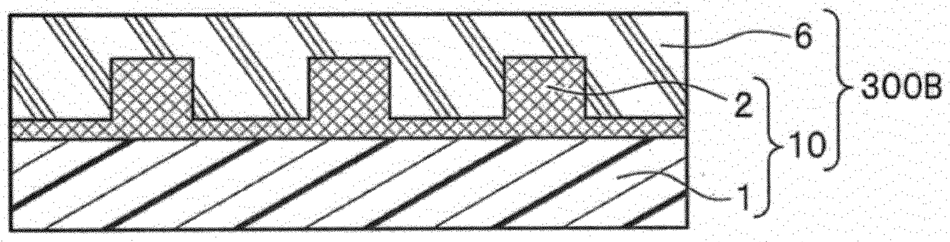


FIG.25

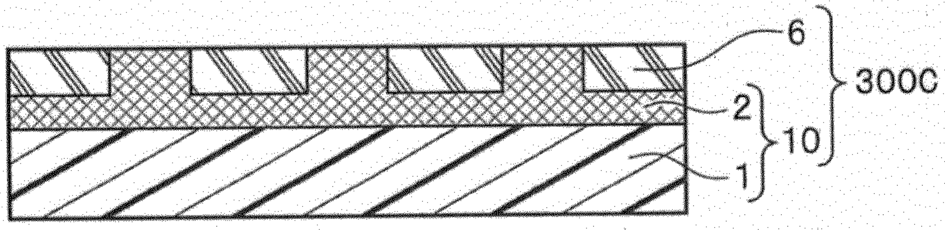

**INTERNATIONAL SEARCH REPORT**

International application No.
PCT/JP2020/008958

A. CLASSIFICATION OF SUBJECT MATTER
see extra sheet

According to International Patent Classification (IPC) or to both national classification and IPC

B. FIELDS SEARCHED

Minimum documentation searched (classification system followed by classification symbols)
C07K7/06; C07K7/08; C07K17/00; A61K9/70; A61L15/22; A61L15/24; A61L15/32; A61L15/44; A61L29/04; A61L29/08; A61L29/12; A61L29/16; A61K47/30; A61K47/32; A61K47/42; A61L31/04; A61L31/10; A61L31/12; A61L31/16; A61L27/14; A61L27/16; A61L27/34; A61L27/40; A61L27/44; A61L27/54

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | 松野寿生, 芹澤武, 疎水性ポリマー膜表面へのタンパク質・ペプチドの吸着固定, Review of Polarography, 2009, vol. 55, no. 1, pp. 5-12, DOI:10.5189/revpolarography. 55.5, page 5, section "Introduction", page 9, section "Short-chain peptides that recognize the polymer surface", etc., (MATSUNO, Hisao, SERIZAWA, Takeshi, "Protein/Peptide Adsorption on Hydrophobic Polymer Material Surfaces") | 1-15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 May 2020 (15.05.2020) | 26 May 2020 (26.05.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2020/008958

C (Continuation). DOCUMENTS CONSIDERED TO BE RELEVANT

| Category* | Citation of document, with indication, where appropriate, of the relevant passages | Relevant to claim No. |
|---|---|---|
| Y | SERIZAWA, Takeshi et al., "A Peptide Motif Recognizing a Polymer Stereoregularity", Journal of the American Chemical Society, 2005, vol. 127, no. 40, pp. 13780-13781, DOI:10.1021/ja054402o, entire text, in particular, table 1, fig. 2 | 1-5 |
| Y | 伊達隆明，芹澤武，高分子結合性ペプチドを用いた機能性ソフト界面の創製，表面科学，2012, vol. 33, no. 1, pp. 21-26, DOI:10.1380/jsssj.33.21, entire text, in particular, page 22, section "2. Identification of PEI-binding peptide", (DATE, Takaaki, SERIZAWA, Takeshi, "Fabrication of Functional Soft Interfaces Using Polymer-Binding Peptides", Hyomen Kagaku) | 1-15 |
| Y | LEUNG, Bonnie O. et al., "Using X-PEEM to study biomaterial s: Prate in and peptide adsorption to a polystyrene-poly(methyl methacrylate)-b-polyacrylic acid blend", Journal of Electron Spectroscopy and Related Phenomena, 2012, vol. 185, pp. 406-416, DOI:10/1016/j.elspec.2012.06.004, abstract, 4.3 Protein and peptide electrostatic interactions | 1-7,12-15 |
| Y | JP 2011-168505 A (KYOTO INSTITUTE OF TECHNOLOGY) 01.09.2011 (2011-09-01) claims, paragraph [0096] | 8-15 |
| Y | JP 2011-517451 A (THE REGENTS OF THE UNIVERSITY OF CALIFORNIA) 09.06.2011 (2011-06-09) claims, paragraph [0032] | 8-15 |
| Y | JP 2018-504167 A (TANGIBLE SCIENCE LLC) 15.02.2018 (2018-02-15) claims | 8-15 |
| A | KUMADA, Yoichi et al., "Screening of PC and PMMA-binding peptides for site-specific immobilization of proteins", Journal of Biotechnology, 2012, vol. 160, pp. 222-228, DOI:10.1016/j.jbiotec.2012.02.010, whole document | 1-15 |
| A | WO 2009/101807 A1 (KYOTO INSTITUTE OF TECHNOLOGY) 20.08.2009 (2009-08-20) entire text | 1-15 |

Form PCT/ISA/210 (continuation of second sheet) (January 2015)

**INTERNATIONAL SEARCH REPORT**
Information on patent family members

International application No.
PCT/JP2020/008958

| Patent Documents referred in the Report | Publication Date | Patent Family | Publication Date |
|---|---|---|---|
| JP 2011-168505 A | 01 Sep. 2011 | (Family: none) | |
| JP 2011-517451 A | 09 Jun. 2011 | US 2011/0046038 A1 claims, paragraph [0041]<br>WO 2009/120893 A2<br>EP 2268673 A2<br>CA 2719250 A<br>KR 10-2011-0005703 A<br>CN 102046661 A | |
| JP 2018-504167 A | 15 Feb. 2018 | US 2017/0360994 A1 claims<br>WO 2016/094533 A1<br>EP 3229851 A1<br>CA 2970010 A<br>CN 107206119 A | |
| WO 2009/101807 A1 | 20 Aug. 2009 | US 2011/0045538 A1 whole document<br>CN 101952432 A | |

Form PCT/ISA/210 (patent family annex) (January 2015)

**INTERNATIONAL SEARCH REPORT**

International application No.
PCT/JP2020/008958

C07K 7/06(2006.01)i; C07K 7/08(2006.01)i; C07K 17/00(2006.01)i; A61K 9/70(2006.01)i; A61L 15/22(2006.01)i; A61L 15/24(2006.01)i; A61L 15/32(2006.01)i; A61L 15/44(2006.01)i; A61L 29/04(2006.01)i; A61L 29/08(2006.01)i; A61L 29/12(2006.01)i; A61L 29/16(2006.01)i; A61K 47/30(2006.01)i; A61K 47/32(2006.01)i; A61K 47/42(2017.01)i; A61L 31/04(2006.01)i; A61L 31/10(2006.01)i; A61L 31/12(2006.01)i; A61L 31/16(2006.01)i; A61L 27/14(2006.01)i; A61L 27/16(2006.01)i; A61L 27/34(2006.01)i; A61L 27/40(2006.01)i; A61L 27/44(2006.01)i; A61L 27/54(2006.01)i

FI: C07K7/06 ZNA; C07K7/08; A61L15/22 100; A61L15/32 100; A61L15/24 100; A61L27/14; A61L27/16; A61L27/34; A61L27/40; A61L29/04; A61L29/08 100; A61L29/12; A61L29/04 100; A61L31/04 110; A61L31/04; A61L31/12; A61L31/10; A61L31/16; A61L31/12 100; A61L29/12 100; A61L29/16; A61L27/54; A61L27/44; A61L15/44 100; A61K9/70 401; A61K47/30; A61K47/42; A61K47/32; C07K17/00

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **TAKESHI SERIZAWA et al.** *Langmuir,* 2007, vol. 23 (22), 11127-11133
- **AKBAR BAGRI et al.** *Nature Chemistry,* 2010, vol. 2, 581-587
- Analysis of Biomolecular Interactions by Biosensor (BIACORETM) Using Surface Plasmon Resonance. **MORIO ARAI.** The Japanese Journal of Thrombosis and Hemostasis. The Japanese Society on Thrombosis and Hemostasis, 01 October 1997, vol. 8, 397-405
- **KEISUKE AIHARA et al.** *Organic Letters,* 2015, vol. 17 (3), 696-699
- Chemical Syntheses of Peptides and Their Applications. **NOBORU YANAIHARA.** Journal of Synthetic Organic Chemistry. The Society of Synthetic Organic Chemistry, 01 November 1998, vol. 46, 1073-1084
- New Technology for Peptide Syntheses. **KIYOSHI NOKIHARA.** KOBUNSHI (in Japanese). The Society of Polymer Science, 01 September 1994, vol. 43, 611-615
- Evaluation of Biocompatibility of Long-term Implantable Device Material in Subcutaneous Implantation Experiment. **TOMOKO SUGIYAMA et al.** JINKO ZOKI (in Japanese. Japan Society for Artificial Organs, 15 April 1998, vol. 28, 509-513

SEQUENCE LISTING

<110> HITACHI, LTD.
TOKYO INSTITUTE OF TECHNOLOGY

<120> Peptide having excellent adhesion to a resin and biocompatible-functional material using the same

<130> 1119001291

<150> JP 2019-071765
<151> 2019-04-04

<160> 33

<170> PatentIn version 3.5

<210> 1
<211> 7
<212> PRT
<213> Artificial Sequence

<220>
<223> Inventor: Iwasaki, Tomio; Maruyama, Masashi; Serizawa, Takeshi; Sawada, Toshiki

<220>
<223> Desingned peptide

<400> 1

Arg Trp Trp Arg Pro Trp Trp
1               5

<210> 2
<211> 7
<212> PRT
<213> Artificial Sequence

<220>
<223> Desingned peptide

<400> 2

Arg Trp Trp Arg Arg Trp Trp
1               5

<210> 3
<211> 7
<212> PRT
<213> Artificial Sequence

<220>
<223> Desingned peptide

<400> 3

Trp Trp Trp Arg Pro Trp Trp
1               5

<210> 4
<211> 7
<212> PRT
<213> Artificial Sequence

<220>
<223> Desingned peptide

<400> 4

Glu Trp Trp Arg Pro Trp Arg
1 5

<210> 5
<211> 7
<212> PRT
<213> Artificial Sequence

<220>
<223> Desingned peptide

<400> 5

Arg Trp Trp Arg Pro Trp Arg
1 5

<210> 6
<211> 12
<212> PRT
<213> Artificial Sequence

<220>
<223> Desingned peptide

<400> 6

Ser Thr Ser Thr Ser Pro Ser Thr Ser Thr Ser Thr
1 5 10

<210> 7
<211> 12
<212> PRT
<213> Artificial Sequence

<220>
<223> Desingned peptide

<400> 7

Thr Ser Thr Ser Thr Pro Thr Ser Thr Ser Thr Ser
1 5 10

<210> 8
<211> 12
<212> PRT
<213> Artificial Sequence

<220>
<223> Desingned peptide

<400> 8

Ser Ser Ser Ser Ser Pro Ser Ser Ser Ser Ser Ser
1 5 10

<210> 9
<211> 12
<212> PRT
<213> Artificial Sequence

<220>
<223> Desingned peptide

<400> 9

Thr Thr Thr Thr Thr Pro Thr Thr Thr Thr Thr Thr
1 5 10

<210> 10
<211> 12
<212> PRT
<213> Artificial Sequence

<220>
<223> Desingned peptide

<400> 10

Ser Thr Ser Thr Ser Thr Ser Thr Ser Thr Ser Thr
1 5 10

<210> 11
<211> 12
<212> PRT
<213> Artificial Sequence

<220>
<223> Desingned peptide

<400> 11

Thr Ser Thr Ser Thr Ser Thr Ser Thr Ser Thr Ser
1 5 10

<210> 12
<211> 12
<212> PRT
<213> Artificial Sequence

<220>
<223> Desingned peptide

<400> 12

Ser Ser Ser Ser Ser Ser Ser Ser Ser Ser Ser Ser
1 5 10

<210> 13
<211> 12
<212> PRT
<213> Artificial Sequence

<220>
<223> Desingned peptide

<400> 13

Thr Thr Thr Thr Thr Thr Thr Thr Thr Thr Thr Thr
1 5 10

<210> 14
<211> 12
<212> PRT
<213> Artificial Sequence

<220>
<223> Desingned peptide

<400> 14

His His His His His His His His His His His His
1 5 10

<210> 15
<211> 12
<212> PRT
<213> Artificial Sequence

<220>
<223> Desingned peptide

<400> 15

His His His His His Pro His His His His His His
1 5 10

<210> 16
<211> 12
<212> PRT
<213> Artificial Sequence

<220>
<223> Desingned peptide

<400> 16

Asp Ser Asp Ser Asp Pro Asp Ser Asp Ser Asp Ser
1 5 10

<210> 17
<211> 12
<212> PRT
<213> Artificial Sequence

<220>
<223> Desingned peptide

<400> 17

Asp Ser Asp Ser Asp Ser Asp Ser Asp Ser Asp Ser
1 5 10

<210> 18
<211> 12
<212> PRT
<213> Artificial Sequence

<220>
<223> Desingned peptide

<400> 18

Asp His Asp His Asp His Asp His Asp His Asp His
1 5 10

<210> 19
<211> 12
<212> PRT
<213> Artificial Sequence

<220>
<223> Desingned peptide

<400> 19

Asp His Asp His Asp Pro Asp His Asp His Asp His
1 5 10

<210> 20
<211> 12
<212> PRT
<213> Artificial Sequence

<220>
<223> Desingned peptide

<400> 20

Asp Glu Asp Glu Asp Pro Asp Glu Asp Glu Asp Glu
1 5 10

<210> 21
<211> 12
<212> PRT
<213> Artificial Sequence

<220>
<223> Desingned peptide

<400> 21

Asp Glu Asp Glu Asp Glu Asp Glu Asp Glu Asp Glu
1 5 10

<210> 22
<211> 12
<212> PRT
<213> Artificial Sequence

<220>
<223> Desingned peptide

<400> 22

Phe His His Phe Phe His His Phe Phe His His Phe
1 5 10

<210> 23
<211> 12
<212> PRT
<213> Artificial Sequence

<220>
<223> Desingned peptide

<400> 23

Phe Phe His His Phe Phe His His Phe Phe His His
1 5 10

<210> 24
<211> 12
<212> PRT
<213> Artificial Sequence

<220>
<223> Desingned peptide

<400> 24

Asn Glu Asn Glu Asn Pro Asn Glu Asn Glu Asn Glu
1 5 10

<210> 25
<211> 12
<212> PRT
<213> Artificial Sequence

<220>
<223> Desingned peptide

<400> 25

Asn Glu Asn Glu Asn Glu Asn Glu Asn Glu Asn Glu
1 5 10

<210> 26
<211> 12
<212> PRT
<213> Artificial Sequence

<220>
<223> Desingned peptide

<400> 26

Val His Phe Pro Thr Lys Ile Ser Glu Gly Asp Met
1 5 10

<210> 27
<211> 12
<212> PRT
<213> Artificial Sequence

<220>
<223> Desingned peptide

<400> 27

Thr Phe Thr Leu Asn Ser Val His Arg Ser Val His
1 5 10

<210> 28
<211> 12
<212> PRT
<213> Artificial Sequence

<220>
<223> Desingned peptide

<400> 28

Ser Pro His Leu His Thr Ser Ser Pro Trp Glu Arg
1 5 10

<210> 29
<211> 12
<212> PRT
<213> Artificial Sequence

<220>
<223> Desingned peptide

<400> 29

Phe Ile Glu Ser Lys Thr Pro Val Asp Pro Asp Gly
1 5 10

<210> 30
<211> 12
<212> PRT
<213> Artificial Sequence

<220>
<223> Desingned peptide

<400> 30

Gly Ser Glu Ser Arg Thr Leu Phe His Pro Glu Gly
1 5 10

<210> 31
<211> 12
<212> PRT
<213> Artificial Sequence

<220>
<223> Desingned peptide

<400> 31

```
Glu Ala Leu Thr Val Asn Ile Lys Arg Glu Met Glu
1               5                   10
```

<210> 32
<211> 12
<212> PRT
<213> Artificial Sequence

<220>
<223> Desingned peptide

<400> 32

```
Ser Met Ile Val Glu Pro Arg Met Leu Ser Thr His
1               5                   10
```

<210> 33
<211> 48
<212> PRT
<213> Bacteriophage M13mp18

<400> 33

```
Ala Glu Gly Asp Asp Pro Ala Lys Ala Ala Phe Asn Ser Leu Gln Ala
1               5                   10                  15

Thr Glu Tyr Ile Gly Tyr Ala Trp Ala Met Val Val Val Ile Val Gly
            20                  25                  30

Ala Thr Ile Gly Ile Lys Leu Phe Lys Lys Phe Thr Ser Lys Ala Ser
        35                  40                  45
```